# EUROPEAN PATENT SPECIFICATION

(11) **EP 0 763 113 B1**
(45) Date of publication and mention of the grant of the patent: **09.03.2005**
(21) Application number: 95916506.9
(22) Date of filing: 21.04.1995
(51) Int. Cl.: C12N 15/29, C12Q 1/68

(54) **GENETIC SEQUENCES CONFERRING DISEASE RESISTANCE IN PLANTS AND USES THEREFOR**
GENETISCHE SEQUENZEN WELCHE PFLANZENKRANKHEITSRESISTENZ VERURSACHEN UND VERWENDUNGEN DAVON
SEQUENCES GENETIQUES CONFERANT A DES PLANTES UNE RESISTANCE AUX MALADIES, ET LEURS UTILISATIONS

(30) Priority: 21.04.1994 AU PM523194; 14.09.1994 AU PM810394
(43) Date of publication of application: 19.03.1997
(73) Proprietor: COMMONWEALTH SCIENTIFIC AND INDUSTRIAL RESEARCH ORGANISATION, Campbell, Australian Capital Territory 2601 (AU)
(72) Inventor: LAWRENCE, Gregory James, Hackett, ACT 2602 (AU); ELLIS, Jeffrey Graham, Weetangera, ACT 2614 (AU); FINNEGAN, Elzabeth Jean, O'Connor, ACT 2601 (AU)
(74) Representative: Owen, Deborah Jane
(86) International application number: PCT/AU1995/000240
(87) International publication number: WO 1995/029238

(56) References cited:
- WO-A-95/18230
- WO-A-95/28423
- WO-A-95/35024
- THEOR. APPL. GENET., 1993, 745-9, XP002040890 MIKLAS, P. N. ET AL: "Identification and potential use of a molecular marker for rust resistance in common bean"
- THEOR APPL GENET, 84 (7-8). 1992. 771-777., XP002040891 XU J ET AL: "TRANSFER OF A DOMINANT GENE FOR POWDERY *MILDEW* *RESISTANCE* AND *DNA* FROM HORDEUM-BULBOSUM INTO CULTIVATED BARLEY HORDEUM-VULGARE"
- SCIENCE, Vol. 265, (23 September 1994), A.F. BENT et al., "RPS2 of Arabidopsis Thaliana: A Leucine-Rich Repeat Class of Plant Disease Resistance Genes", pages 1856-1860.
- SCIENCE, Vol. 266, (4 November 1994), P.A. JONES et al., "Isolation of the Tomato Cf.9 Gene for Resistance to Cladosporium Fulvum by Transposon Tagging", pages 789-793.
- CELL, Volume 78, (23 September 1994), S. WHITHAM et al., "The Product of the Tobacco Mosaic Virus Resistance Gene N: Similarity to Toll and the Interleukin-1 Receptor", pages 1101-1115.
- BIO/TECHNOLOGY, Volume 11 (9), (September 1993), E. TRUVE et al., "Transgenic Potato Plants Expressing Mammalian 2'-5' Oligoadenylate Synthetase are Protected from Potato Virus X Infection under Field Conditions", pages 1048-1052.
- SCIENCE, Volume 258, (6 November 1992), G.S. JOHAL and S.P. BRIGGS, "Reductase Activity Encoded by the HM1 Disease Resistance Gene in Maize", pages 985-987.
- SCIENCE, Volume 262, (26 November 1993), G.B. MARTIN et al., "Map-Based Cloning of a Protein Kinase Gene Conferring Disease Resistance in Tomato", pages 1432-1436.
- ADVANCES IN MOLECULAR GENETICS OF PLANT-MICROBE INTERACTIONS, Volume 14, (1993), A. PRYOR, "Transposon Tagging of a Rust Resistance Gene in Maize", pages 469-475.
- ADVANCES IN MOLECULAR GENETICS OF PLANT-MICROBE INTERACTIONS, Volume 14, (1993), H.J. NEWBURY et al., "Mutagenesis of a Race-Specific Rust Resistance Gene in Antirrhinum Majus Using a Transposon-Tagging Protocol", pages 511-515.
- CELL, Volume 80, (10 February 1995), J.L. DANGL, "Piece de Resistance: Novel Classes of Plant Disease Resistance Genes", pages 363-366.

## Description

The present invention relates generally to genetic sequences, and more particularly to genetic sequences which confer or otherwise facilitate disease resistance in plants such as against rust and mildew. The present invention further provides for transgenic plants carrying the subject genetic sequences enabling the generation of disease resistant plants. The present invention is particularly useful for developing disease resistance in crop varieties.

Bibliographic details of the publications numerically referred to in this specification are collected at the end of the description. Sequence Identity Numbers (SEQ ID NOs.) for the nucleotide and amino acid sequences referred to in the specification are defined following the bibliography.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

The rapidly increasing sophistication of recombinant DNA technology is greatly facilitating the efficacy of commercially important agricultural processes. Of particular concern is the effect of plant diseases on the efficacy of these agricultural processes. Plant diseases and particularly diseases in crop plants represent a major contributing factor in crop losses capable of causing economically significant down turn in productivity per square metre. The development, therefore, of disease resistant plants is an important goal in agricultural and horticultural research.

Major genes conditioning resistance to plant diseases have been investigated extensively in agriculture. Of particular economic importance are genes controlling resistance to rust and mildew. Rust is an especially significant problem amongst broad acre crops such as wheat, barley and cereal grains and is caused by infection with a class of fungi known as the *Basidiomycetes.* Although rust resistance genes are a potentially invaluable genetic resource in agriculture, the molecular basis of major gene resistance is unknown and, until the present invention, genes conferring rust or mildew resistance had not been cloned.

Genetic analysis indicates that rust resistance genes control specific recognition of the products of rust avirulence genes. In developing disease resistance varieties using disease resistance genes, plant breeders deploy resistance genes that match the avirulence genes present in the local strains of the pathogens. The pathogen populations are dynamic and frequently new pathogenic strains arise by any number of means such as by mutation, recombination or accidental or natural introduction of new pathogenic strains. The existing disease resistant varieties then become susceptible to the new pathogenic strains. Plant breeders are then forced, to develop new disease resistant varieties. At present, breeders use resistance genes that exist in either wheat or its relatives as their pool of new genes.

The cloning of a resistance gene is the first step in understanding the basis of resistance gene action and in particular the specificity control mechanism. Ellis *et al* (1) have described the development of a transposon tagging system to isolate rust resistance genes from flax (linseed, *Linum usitatissimum).* However, despite the general effectiveness of the methodology, the isolation of mutant flax plants possessing a tagged resistance gene has not been a straight forward procedure (2). Miklas *et al* (15) describes the identification of a molecular marker of rust resistance and its use as a selection tool.

In accordance with the present invention, genetic sequences conferring rust resistance have been cloned from flax. The cloning of these sequences provides the means of generating transgenic plants with *de novo,* increased or otherwise enhanced rust resistance. The present invention also permits the screening through genetic or immunological means similar rust resistance genes in other plants for use in developing or enhancing rust resistance in commercially and economically important species. Furthermore, the application of knowledge of the molecular basis behind resistance gene action and the specificity thereof offers the potential of a new source of genes produced by a variety of recombinant techniques. These new genes with altered disease resistance specificites are referred to herein as "modular resistance genes".

Accordingly, one aspect of the present invention comprises an isolated nucleic acid molecule comprising a sequence of nucleotides which confers or otherwise facilitates disease resistance in a plant. More particularly, the disease resistance is rust resistance. The present invention extends, however, to resistance to obligate biotrophic pathogens including but not limited to fungi, viruses and nematodes.

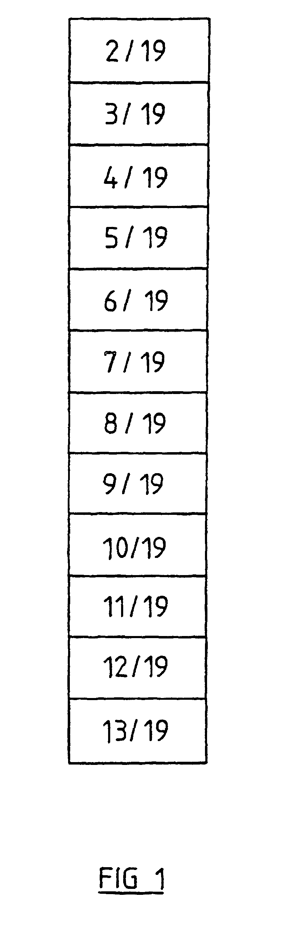

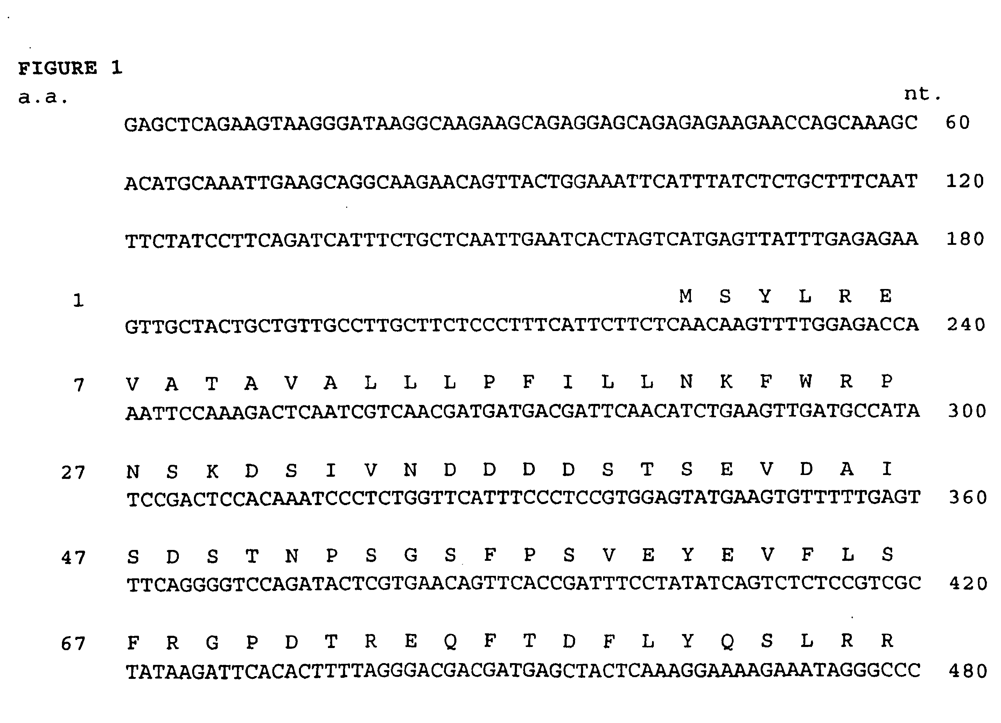

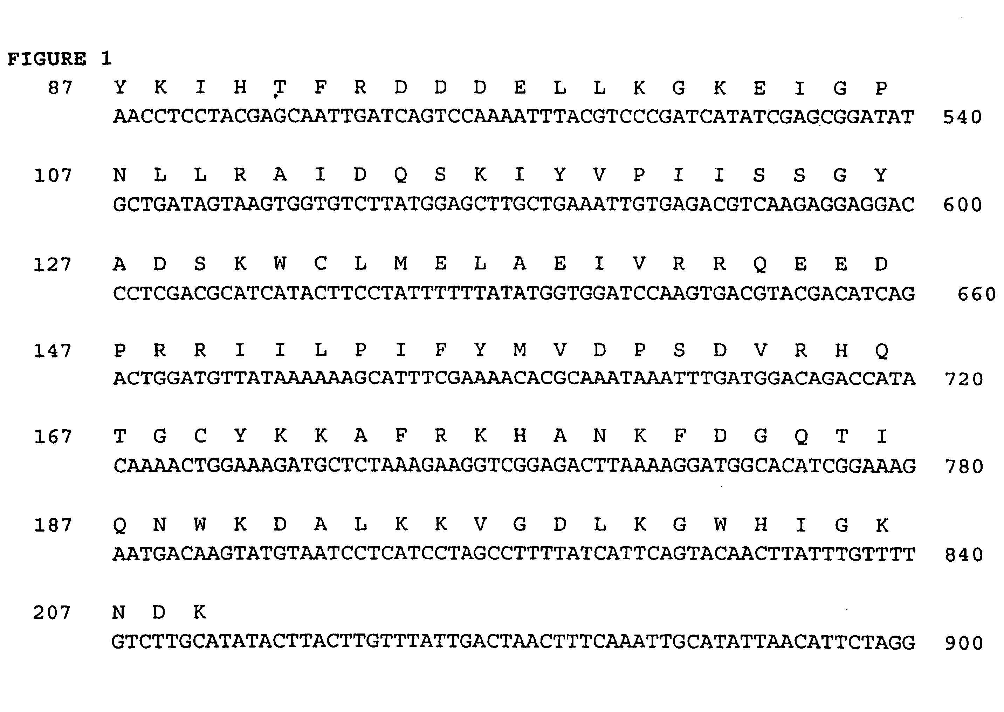

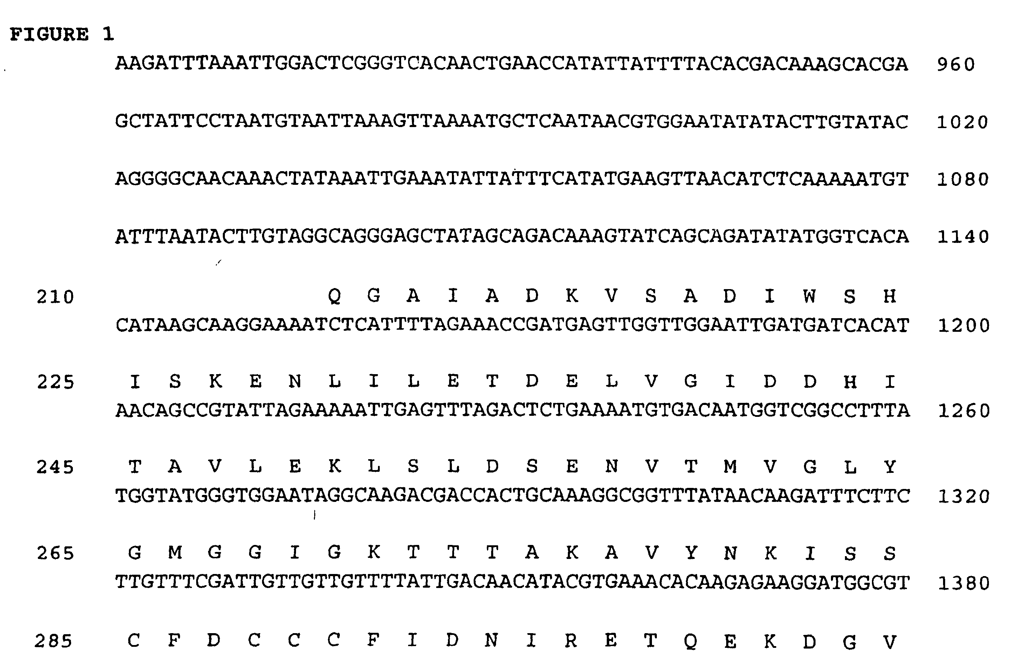

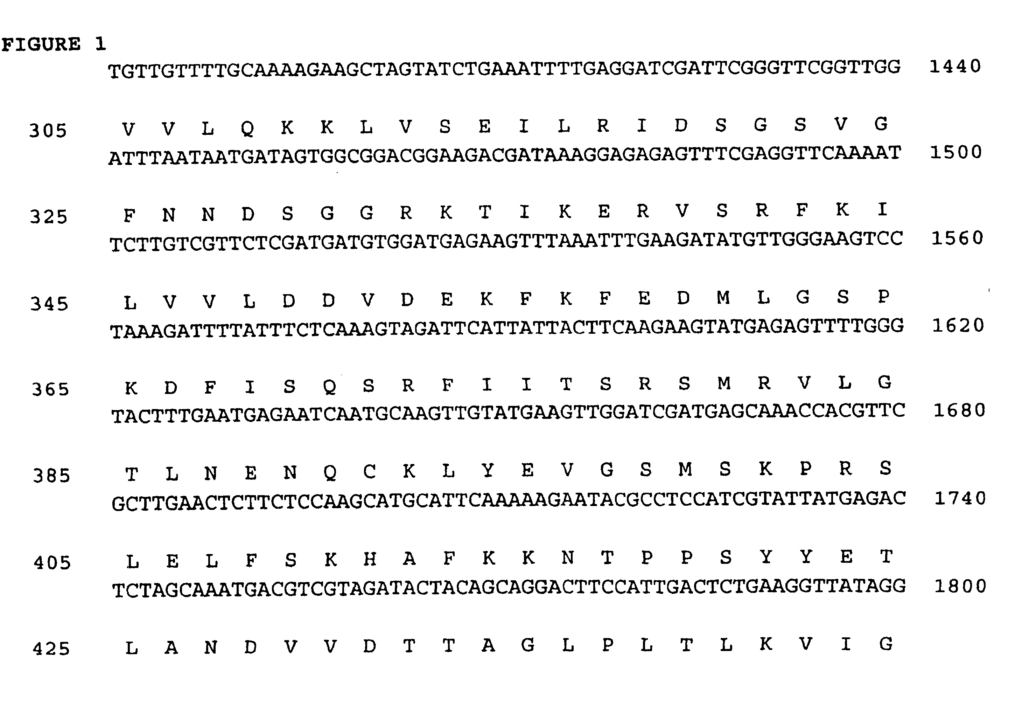

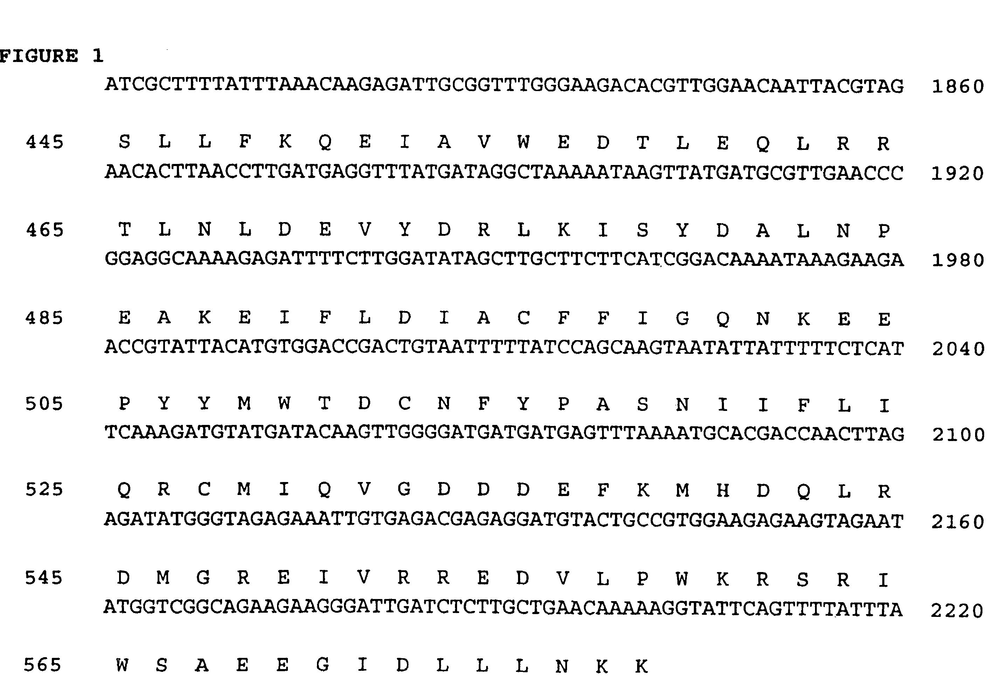

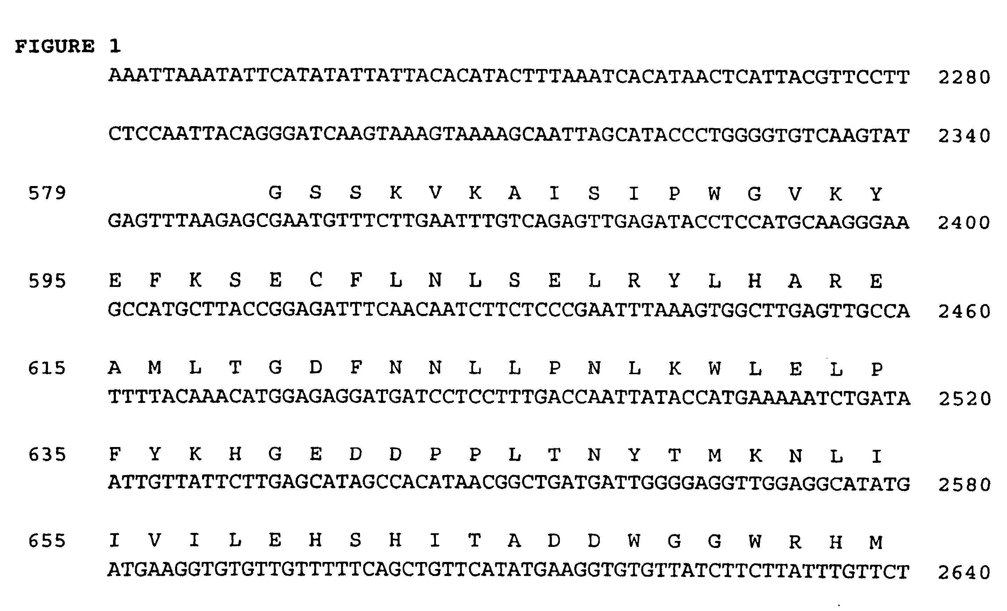

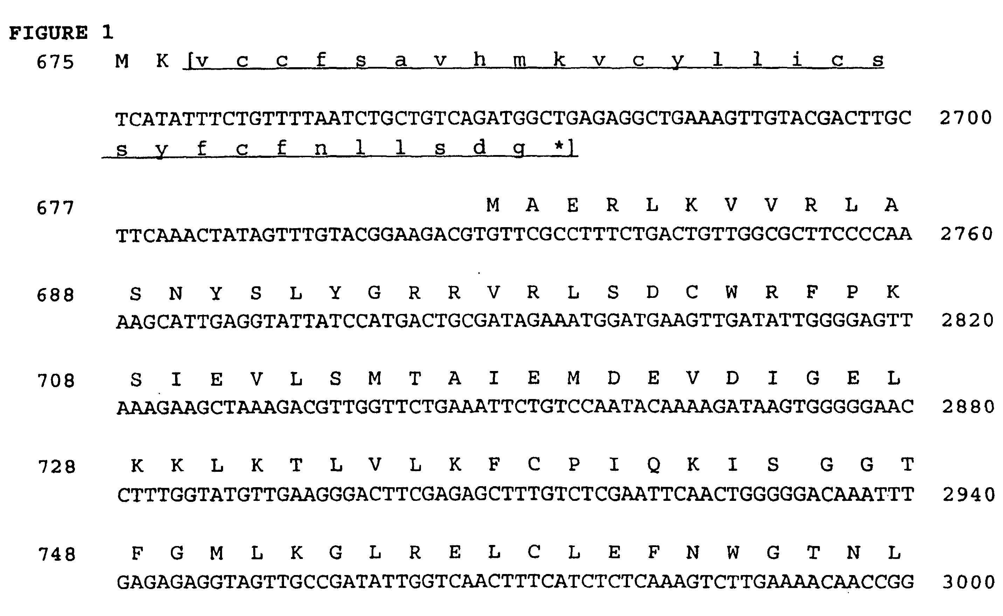

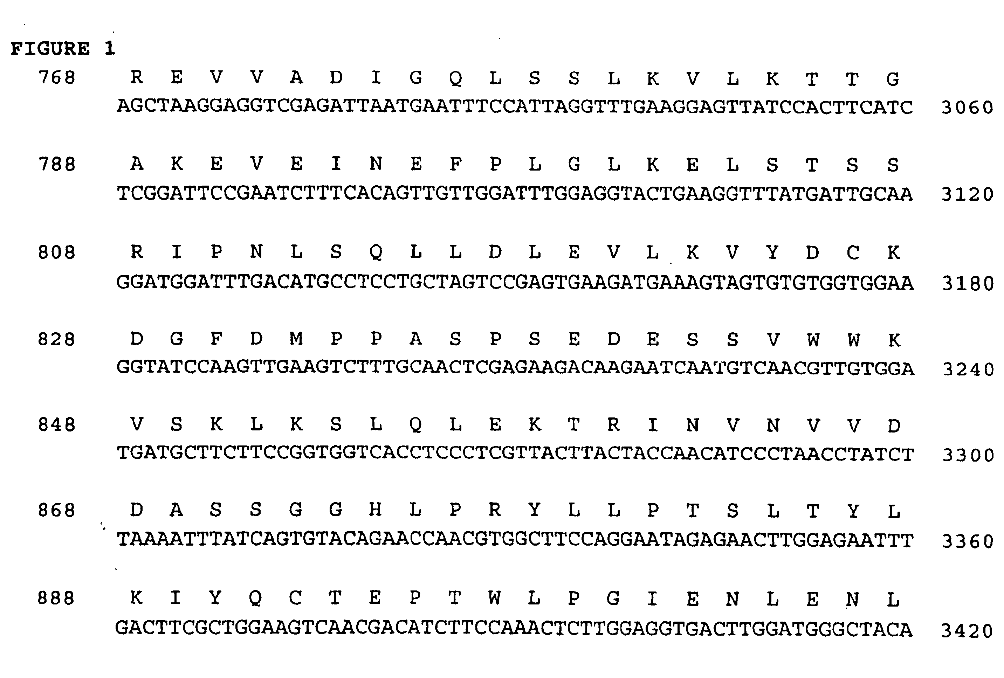

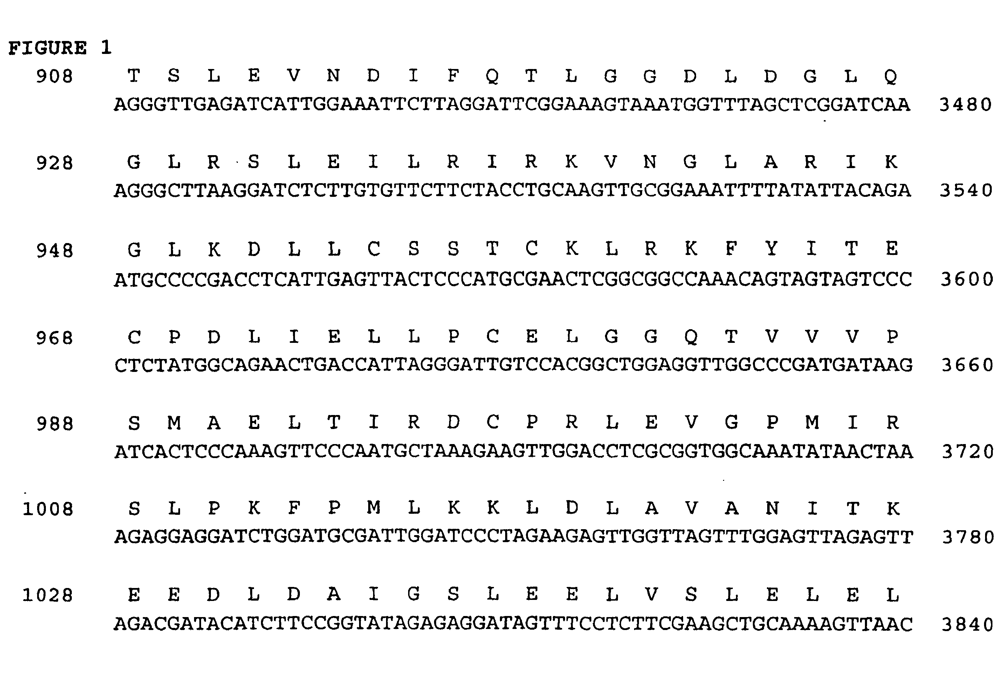

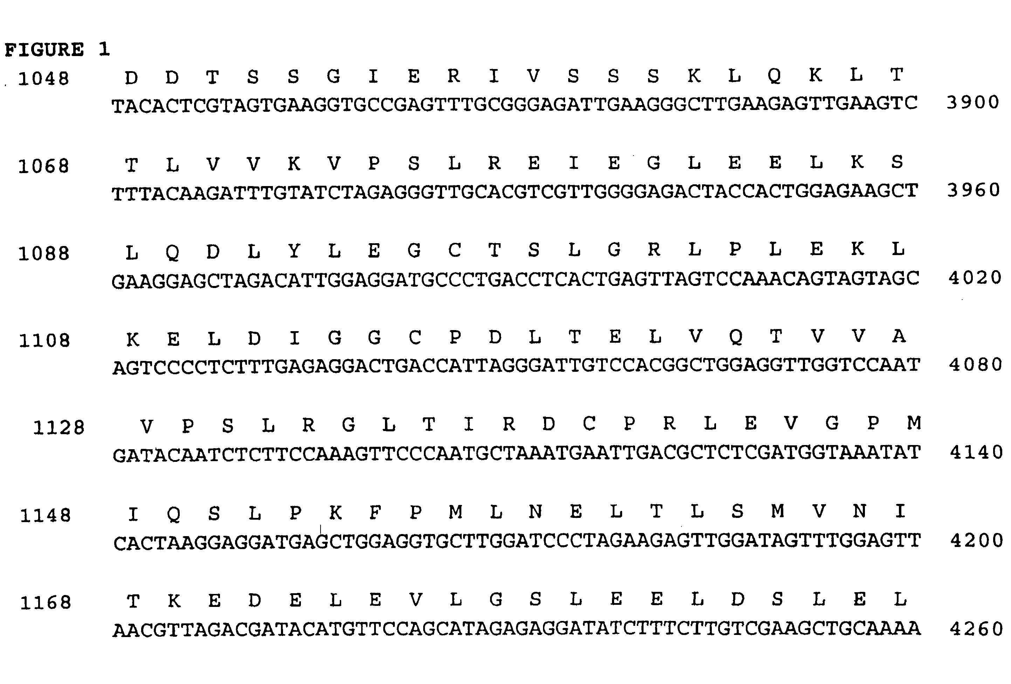

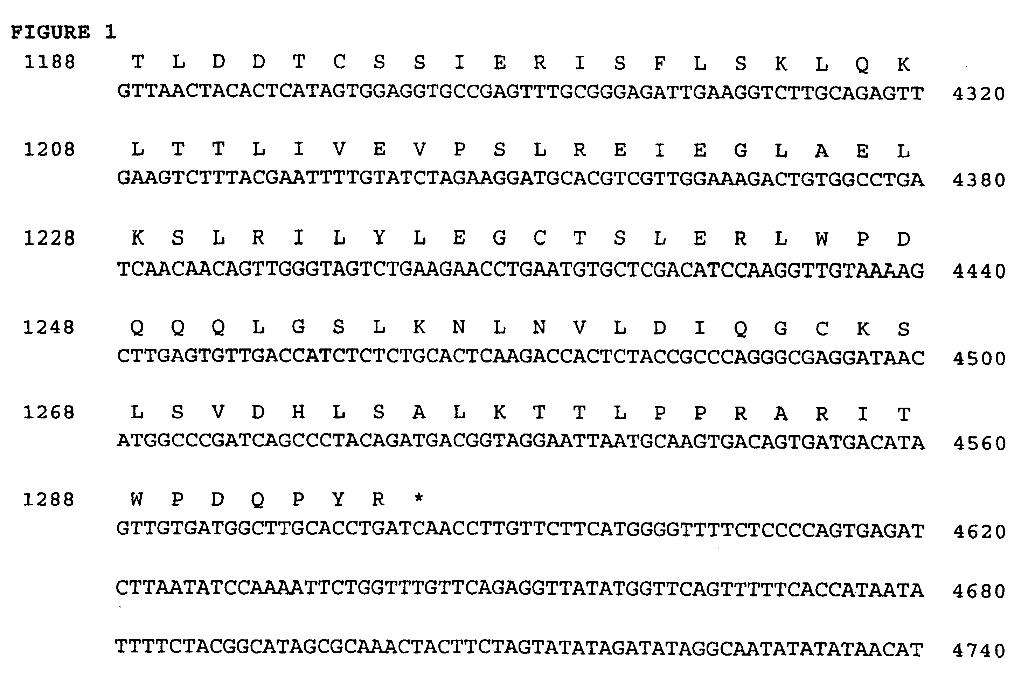

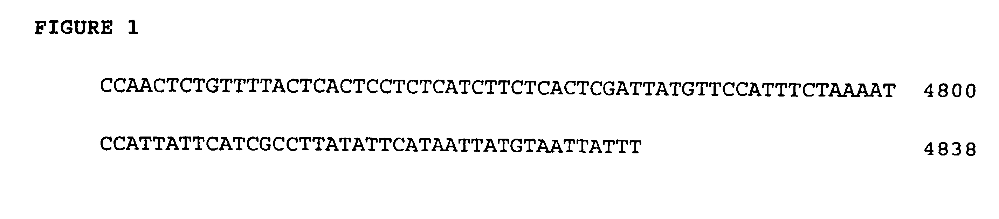

Another aspect of the present invention is directed to a nucleic acid molecule which comprises a sequence of nucleotides corresponding or complementary to the nucleotide sequence set forth in Figure 1 (SEQ ID NO:1) or having at least 45% similarity to all thereof and wherein said nucleic acid molecule confers or otherwise facilitates rust resistance in a plant.

In a related aspect of the present invention there is provided a nucleic acid molecule which comprises a sequence of nucleotides encoding or complementary to a sequence of nucleotides encoding the amino acid sequence set forth in Figure 1 (SEQ ID NOs:2 to 5) or having at least 60% similarity to all thereof and wherein said nucleic acid molecule confers or otherwise facilitates rust resistance in a plant.

Preferably, the percentage similarity is at least 65%. Yet still more preferably, the percentage similarity is at least 80-90% including at least 91% or 93% or 95%.

For the purposes of nomenclature, the sequences in Figure 1 relate to the "L6" allele of the disease resistance gene L which controls host resistance to flax rust races that carry the corresponding avirulence gene AL6. However, the present invention extends to other alleles of the gene or alleles of the M gene. Accordingly, a related embodiment of the present invention contemplates a nucleic acid molecule comprising a sequence of nucleotides encoding or complementary to a sequence encoding a peptide, polypeptide or protein wherein said peptide, polypeptide or protein is capable of interacting with an avirulence gene product on a flax rust. Generally, when the rust resistance gene is the L6 allele, the corresponding avirulence gene is AL6. A similar nomenclature applied to the other alleles of the L gene, for example, the L2 and L10 alleles. Their corresponding avirulence genes are AL2 and AL10, respectively.

A further aspect of the present invention contemplates a nucleic acid molecule which confers or otherwise facilitates rust resistance in a plant and which is capable of hybridising under at least medium stringency conditions to the nucleic acid molecule defined in SEQ ID NO: 1.

For the purposes of defining the level of stringency, reference can conveniently be made to Sambrook *et al* (3), where the washing steps at pages 9.52-9.57 are considered high stringency. A "low" stringency is defined herein as being in 0.1-0.5% w/v SDS at 37-45°C for 2-3 hours.
Depending on the source and concentration of nucleic acid involved in the hybridisation, alternative conditions of stringency may be employed such as "medium" stringent conditions which are considered herein to be 0.25-0.5% w/v SDS at ≥45°C for 2-3 hours or "high" stringent conditions as disclosed by Sambrook *et al* (3).

It is proposed that the genetic sequences of the present invention are necessary for specific recognition of the products of rust avirulence genes. Accordingly, the genetic sequences are useful in enhancing existing rust resistance genes, providing *de* *novo* the required specific recognition of rust avirulence gene products or being introduced together with rust avirulence genes on, for example, a single genetic cassette. Accordingly, these aspects of the invention are covered by the expression "conferring or otherwise enhancing rust resistance" or other similar expression.

The present invention is particularly directed to resistance conferring or facilitating genetic sequences from flax plants and from its relative *Linum marginale.* However, the subject invention clearly contemplates other sources of rust resistance genes such as but not limited to other species of *Linum,* soybeans, sunflowers and cereals amongst other plants including wild varieties of wheat, barley and maize.

The genetic sequences conferring or otherwise facilitating rust resistance may correspond to the naturally occurring sequence or may differ by one or more nucleotide substitutions, deletions and/or additions. Accordingly, the present invention extends to rust resistance genes and any functional alleles mutants, derivatives, parts, fragments, homologues or analogues thereof or non-functional molecules but which are at least useful as, for example, genetic probes or in the generation of immunologically interactive recombinant molecules. The present invention further extends to the promoter region from the rust resistance genes such as from L6, L2 or L10. A preferred promoter is from L6. Such promoters may be useful in driving expression of transgenes.

Examples of alleles of the flax rust resistance gene L include, but are not limited to L6, L2 and L10 although all alleles of the L locus are encompassed by the present invention. The present invention also extends to alleles of the M locus.

The present invention further contemplates recombinant or synthetic rust resistance gene products, such as the products of the L6, L2 or L10 alleles of the L resistance gene. A particularly preferred recombinant or synthetic rust resistance gene product is from the L6 gene. The L6 protein has the following features; an amino terminal half containing a nucleotide binding site (NBS) consisting of several motifs including the P-loop and further downstream, a kinase-2 motif (14). In L6, the kinase-2 sequence is ILVVLDDVD (SEQ ID NO: 13) and more generally, XXXXDDX where X=L,I,V,F (using single amino acid nomenclature defined below). The C-terminal half of the polypeptide is a leucine rich region consisting of approximately 17% leucine residues. Within this region, stretches of residues appear where leucine is reiterated LXXL or LXL where X = any residue. These sequences are similar to the leucine-rich repeat sequences of 25-30 residues that are found in many proteins that form protein-protein interactions. More particularly in the L6 product, the leucine rich region of L6 consists of two parts with the C-terminal part of the region consisting of two direct leucine rich repeats of 146 and 149 amino acids that are 74% identical.

The above mentioned functional mutants, derivatives, parts, fragments, homologues and analogues of are referred to herein as "rust resistance-like genes" or "rust resistance genetic sequences". Reference herein to "genes" is to be taken in its broadest context and includes a classical genomic gene as well as mRNA or cDNA corresponding to the coding regions (i.e. exons) of the gene. The term "gene" is also used to describe synthetic or fusion molecules encoding all or part of a functional product. Preferred rust resistance-like genes are derived from a naturally occurring rust resistance gene by standard recombinant techniques. Generally, a rust resistance gene may be subjected to mutagenesis to produce single or multiple nucleotide substitutions, deletions and/or additions. Nucleotide insertional derivatives of the rust resistance gene of the present invention include 5' and 3' terminal fusions as well as intra-sequence insertions of single or multiple nucleotides. Insertional nucleotide sequence variants are those in which one or more nucleotides are introduced into a predetermined site in the nucleotide sequence although random insertion is also possible with suitable screening of the resulting product. Deletional variants are characterised by the removal of one or more nucleotides from the sequence. Substitutional nucleotide variants are those in which at least one nucleotide in the sequence has been removed and a different nucleotide inserted in its place. Such a substitution may be "silent" in that the substitution does not change the amino acid defined by the codon. Alternatively, substituents are designed to alter one amino acid for another similar acting amino acid. Typical substitutions are those made in accordance with the following:

| Suitable residues for amino acid substitutions | |
|---|---|
| Original Residue | Exemplary Substitutions |
| Ala | Ser |
| Arg | Lys |
| Asn | Gln; His |
| Asp | Glu |
| Cys | Ser |
| Gln | Asn |
| Glu | Asp |
| Gly | Ala |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

In a particularly preferred embodiment, the rust resistance genetic sequences or like genetic sequences are employed to identify and isolate similar, genes from other plants. According to this embodiment, there is contemplated a method for identifying a rust resistance genetic sequence or rust resistance-like genetic sequence in a plant, said method comprising contacting genomic DNA or cDNA isolated from said plant with a hybridisation effective amount of a genetic sequence conferring or otherwise rust resistance or part thereof and then detecting said hybridisation.

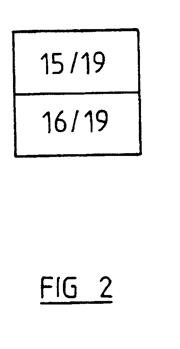

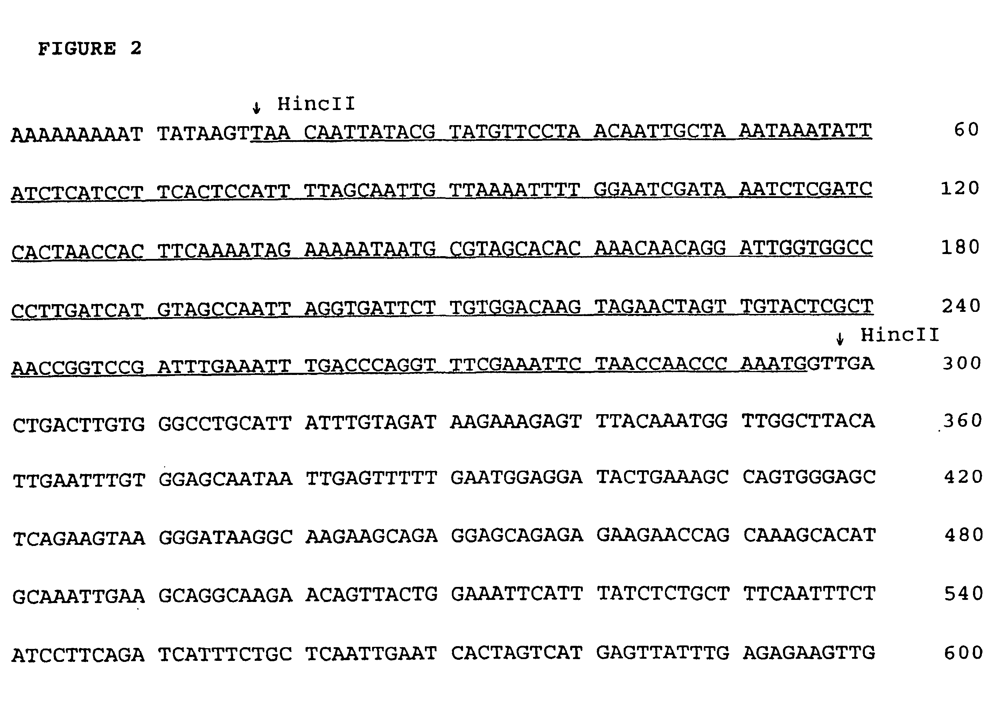

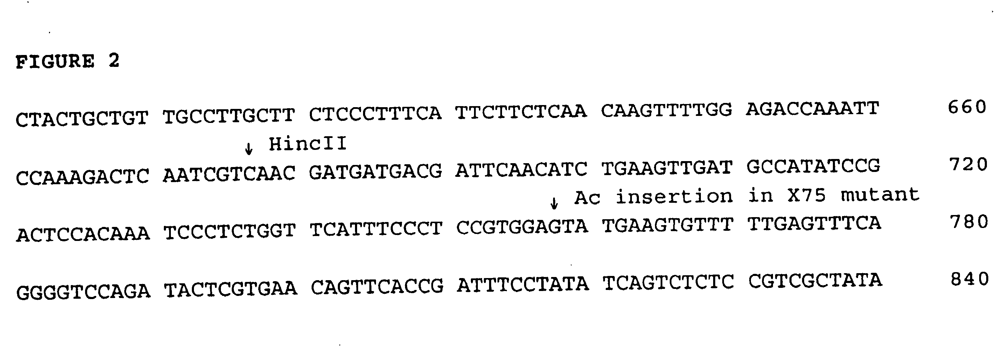

Preferably, the latter mentioned genetic sequence is from flax or similar plant such as a *Linum* species. In a most preferred embodiment, the latter genetic sequence is as set forth in SEQ ID NO:1 or corresponds to a probe such as defined in Figure 2 (SEQ ID NO:11).

Preferably, the latter genetic sequence is labelled with a reporter molecule capable of giving an identifiable signal (e.g. a radioisotope such as ³²P or ³⁵S or a biotintylated molecule).

Preferably, the plant to be screened is a flax, a Linum species or a grain crop plant such as wheat, barley, maize, rye, lupins or rice and/or wild varieties of same.

Alternatively, the plant is screened using antibodies to a recombinant product of a rust resistance gene. According to a first aspect of this embodiment, the present invention provides for the expression of the subject genetic sequence in a suitable host (e.g. a prokaryote or eukaryote) to produce full length or non-full length recombinant rust resistance gene products. The only requirement being that the recombinant products are immunologically interactive with antibodies to all or part of a rust resistance gene product. The immunological screening for rust resistance gene products may, in some circumstances, be more suitable than a genetic screening procedure.

Another aspect is, therefore, directed to antibodies to a recombinant rust resistance gene product or part or fragment thereof. Such antibodies may be monoclonal or polyclonal and may be selected from naturally occurring antibodies to a rust resistance gene product or may be specifically raised to a recombinant rust resistance gene product. In the case of the latter, the rust resistance gene product may first need to be associated with a carrier molecule. Alternatively, fragments of antibodies may be used such as Fab fragments. Furthermore, recombinant and synthetic antibodies and antibody hybrids are described. A "synthetic antibody" is considered herein to include fragments and hybrids of antibodies. In additions, antibodies may be raised to fragments or derivatives of the rust resistance gene product such as oligopeptides derived from or based on the product of, for example, the L6 gene. The antibodies and/or the recombinant rust resistance gene products are particularly useful for the immunological screening of rust resistance gene products in various plants, in monitoring expression of rust resistance genetic sequences in transgenic plants and as a proprietary tagging system.

In one embodiment, specific antibodies are used to screen for rust resistance gene products in plants. Techniques for the assays contemplated herein are known in the art and include, for example, sandwich assays and ELISA.

It is possible to include any second antibodies (monoclonal, polyclonal or fragments of antibodies) directed to the first mentioned antibodies discussed above. Both the first and second antibodies may be used in detection assays or a first antibody may be used with a commercially available anti-immunoglobulin antibody. An antibody as contemplated herein includes any antibody specific to any region of a recombinant rust resistance gene product.

Both polyclonal and monoclonal antibodies are obtainable by immunisation with a recombinant rust resistance gene product and either type is utilisable for immunoassays. The methods of obtaining both types of sera are well known in the art. Polyclonal sera are less preferred but are relatively easily prepared by injection of a suitable laboratory animal with an effective amount of recombinant rust resistance gene product, or antigenic or immunointeractive parts thereof, collecting serum from the animal and isolating specific sera by any of the known immunoadsorbent techniques. Although antibodies produced by this method are utilisable in virtually any type of immunoassay, they are generally less favoured because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitised against the immunogenic preparation can be done by techniques which are well known to those who are skilled in the art (see, for example, references 4, 5 and 6).

The presence of a rust resistance gene product in a plant or more commonly plant extract may be accomplished in a number of ways such as by Western blotting and ELISA procedures. A wide range of immunoassay techniques are available as can be seen by reference to US Patent Nos. 4,016,043, 4, 424,279 and 4,018,653. These, of course, includes both single-site and two-site or "sandwich" assays of the non-competitive types, as well as in the traditional competitive binding assays. These assays also include direct binding of a labelled antibody to a target.

Sandwich assays are among the most useful and commonly used assays and are favoured for use in the present invention. A number of variations of the sandwich assay technique exist. Briefly, in a typical forward assay, an unlabelled antibody is immobilised on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule.

In this case, the first antibody is raised to a recombinant rust resistance gene product and the antigen is a rust resistance gene product in a plant.

The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody.

These techniques are well known to those. skilled in the art, including any minor variations as will be readily apparent. The sample is one which might contain rust resistance gene product and include crude or purified plant extract such as extracts of leaves, roots and stems.

In the typical forward sandwich assay, a first antibody raised against a recombinant rust resistance gene product is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking, covalent binding or physically adsorption, the polymer-antibody complex is washed in preparation for the test sample. An aliquot of the sample to be tested is then added to the solid phase complex and incubated for a period of time sufficient (e.g. 2-40 minutes) and under suitable conditions (e.g. 25°C) to allow binding of any antigen present in the sample to the antibody. Following the incubation period, the reaction locus is washed and dried and incubated with a second antibody specific for a portion of the first antibody. The second antibody is linked to a reporter molecule which is used to indicate the binding of the second antibody to the hapten.

An alternative method involves immobilising the target molecules in the biological sample and then exposing the immobilised target to specific antibody which may or may not be labelled with a reporter molecule. Depending on the amount of target and the strength of the reporter molecule signal, a bound target may be detected by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognised, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates which yield a fluorescent product rather than the chromogenic substrates noted above. In all cases, the enzyme-labelled antibody is added to the first antibody-hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of hapten which was present in the sample. The term "reporter molecule" also extends to use of cell agglutination or inhibition of agglutination such as red blood cells on latex beads, and the like.

Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. As in enzyme immunoassays (EIA), the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

It will be readily apparent to the skilled technician how to vary the above assays and all such variations are encompassed by the present invention.

The present invention is particularly described with reference to the flax rust resistance gene L and its alleles L6, L2 and L10. This is done, however, with the understanding that the subject invention extends to a range of resistance genes and alleles thereof for rust and other pathogens. In fact, the present invention extends to a resistance gene characterised by said gene encoding a product having at least one leucine rich region. Preferably, the leucine rich region is a leucine rich repeat at the 3' end of the molecule and has at least 60% similarly to each other more preferably at least 70% similarity and even more preferably at least 80% similarity. Still more preferably, the leucine rich region corresponds to the following amino acid sequence: or having at least 60% similarly thereto or more preferably at least 70% similarity or even more preferably at least 80% similarity thereto. Alternatively or in addition to, the rust resistance gene further encodes a p-Loop at its 5' end encoding the amino acid sequence: where X is any amino acid residue. More preferably, the p-Loop sequence comprises the amino acid residues: and more particularly or having one or more amino acid substitutions, insertions and/or deletions thereto provided that such derivatives still function as a p-Loop. A p-Loop is involved in ATP/GTP binding. It is proposed herein that copy number and amino acid sequence of this repeated element are the determinants of the gene-for-gene specificity of rust resistance genes. Mixing and matching of these elements from existing genes will provide a potential means for creating new and useful resistance gene specificities for use in plant breeding. Such new genes are referred to herein as "modular resistance genes". This is a completely novel approach to disease resistance breeding.

According to this embodiment, there is contemplated a modular resistance gene characterised in that said gene encodes a non-naturally occurring leucine rich region. By "non-naturally occurring" is meant to include the manipulation of a genetic sequence to introduce a leucine rich encoding sequence, to increase the copy number of an existing leucine rich encoding sequence, to delete or insert a nucleotide sequence for or into a leucine rich encoding sequence or to otherwise modify a leucine rich encoding sequence to modulate pathogen specificity of a disease resistance gene. For example, the leucine rich regions of two or more of alleles of the L locus such as L6, L2 and L10 could be combined to broaden the range of pathogens to which a gene can confer resistance. Alternatively, combinations of allels from the L and M loci could be made. The present invention contemplates, therefore, a method of producing a modular resistance gene conferring resistance to a pathogen in a plant by manipulating the leucine rich regions in a disease resistance gene.

The present invention further extends to transgenic plants such as transgenic crop plants (e.g. wheat, barley or maize) carrying a non-indigenous genetic sequence conferring or otherwise facilitating rust resistance in said plant. Preferably, the non-indigenous genetic sequence is from a closely related species to the transgenic plant. The expression of the non-indigenous genetic sequence may be constitutive or inducible or developmentally regulated. Furthermore, the non-indigenous sequence may be inserted into or fused to a particular endogenous genetic sequence. In a most preferred embodiment, the transgenic plant is wheat and the non-indigenous genetic sequence is from a wild variety of wheat, barley, maize, flax or *Linum* species. Other transformed species or resistance gene sources are not excluded.

The present invention is further described by reference to the following non-limiting Figures and Examples.

In the Figures:
**Figure 1** is a representation of the nucleotide sequence (SEQ ID NO: 1) and corresponding amino acid sequence (SEQ ID NOs:2 to 5) of the rust resistance gene L6. There are two predicted products, the "full length" and "truncated" products. The truncated product results from an alternately spliced mRNA that retains intron 3. In the absence of the splicing of this intron, translation would continue through the 82bp intron 3 as a consequence of not changing frames, a stop codon is reached just downstream of the end of the intron. This results in a product from which the major portion of the leucine rich region is removed and also the addition of an extra 29 amino (see underlined amino acids [SEQ ID NOs: 6 and 7) that are not in the full length product.
**Figure 2** is a representation of part of the L6 gene showing the LU-1 probe (underlined) and Ac insertion in mutant X75 (SEQ ID NO:11).
**Figure 3** is a schematic representation of the L6 gene showing location of LU-2 probe.
**Figure 4** is a representation of the amino acid sequences of the leucine rich repeat in L6.
**Figure 5** is a schematic representation of the L locus alleles.

### EXAMPLE 1

### PLANT MATERIAL

Experiments were conducted in the line of flax called "Forge" which is homozygous for the rust resistance genes L⁶, M, N and P² (2). One particular flax is designated TC257 and is a primary transformant of "Forge" possessing 10 copies of the maize transposable element Ac (I). Rust gene cultivars are referred to as "Birio" (L⁶), "Dakota" (M), "Bombay" (N) and "Abyssinian" (P²) (8).

### EXAMPLE 2

### RUST STRAINS

Four rust strains were used to screen for mutants. Three of these strains, designated CHS-78, CH5-84 and CH5-133, were obtained by selfing strain CH5. The fourth strain designated C was a parent strain of CH5. The origins of C and CH5 have been previously described (7). Each of these strains is avirulent on one of the single gene differentials and virulent on the other three. Strain CH5-84 is avirulent on "Birio" (L⁶), CH5-78 is avirulent on "Dakota" (M), C is avirulent on "Bombay" (N) and CH5-133 is avirulent on "Abyssinian" (P²). Rust maintenance and inoculation procedures were as previously described (8).

### EXAMPLE 3

### TRANSFORMATION

Flax cotyledons were transformed with either the vector pKU3 (9), pBT175 (10), pB135SAc11, 12 (11) or an Ac element in which the OCS enhancer (12) had been inserted at the BamH1 site at position 182, according to the protocol previously described (13).

### EXAMPLE 4

### TAGGING SCHEME

The tagging scheme used in this study has been described by Ellis *et al* (1) and Lawrence *et al* (13). "Forge" plants with and without Ac elements, including TC257 and its selfed progeny, were extensively crossed as the female parent with "Hoshangabad" to produce hybrid seed heterozygous for the four rust resistance genes. When seedlings were about 5cm tall, the tip was cut off and the two lateral shoots that subsequently developed were inoculated with a mixture of the four rust strains, each of which recognise a single resistance gene. Most plants were resistant to all four rust strains. Susceptible mutants were of three types, namely, "whole", in which all leaves on both lateral shoots were susceptible, "bisectored", in which all leaves on one lateral were susceptible while the other shoot was entirely resistant and "mini-sectored", in which only some of the leaves on one shoot were susceptible. When rare rust-susceptible mutants were detected, the mutant gene was identified by recovering rust spores and inoculating these on to a set of four rust resistance genes L⁶ , M, N or P². If, for example, the rust recovered from a susceptible mutant plant grew on the M, N and P² differentials but not on the L⁶ differential, then this indicated that the plant was mutant for the L⁶ gene.

### EXAMPLE 5

### DETECTION OF TRANSPOSED Ac ELEMENTS IN MUTANT PLANTS

The procedure for identifying transposed Ac elements was as previously described (2).

### EXAMPLE 6

### LAMBDA CLONING

Flax DNA was digested with *Bam*Hl or *Bgl*II and the unfractionated DNA was cloned into the Lambda vector EMBL4 as previously described (3).

### EXAMPLE 7

### GENETIC ANALYSIS

Genetic analysis including RFLP techniques, PCR analysis, Southern blot analysis and linkage analysis were as previously described (1; 2). The nucleotide and corresponding amino acid sequence for the L6 gene is shown in Figure 1. There are two predicted products, the "full length" and "truncated" products. The truncated product results from an alternately spliced mRNA that retains intron 3. In the absence of the splicing of this intron, translation would continue through the 82bp intron 3 and as a consequence of not changing frames, a stop codon is reached just downstream of the end of the intron. This results in a product from which the major portion of the leucine rich region is removed and also the addition of an extra 29 amino acids (see underlined amino acids in Figure 1) that are not in the full length product.

### EXAMPLE 8

### IDENTIFICATION AND CHARACTERISATION OF A RUST-SUSCEPTIBLE MUTANT PLANT IN WHICH THE L6 GENE HAS BEEN TAGGED BY Ac

The TC257 (Example 1) line of transgenic flax containing at least 10 copies of Ac (1; 2), one of which was linked (29 map units) from the L6 rust resistance gene, was crossed extensively to a line without resistance genes and the progeny were screened for lack of L6 activity. One rust susceptible mutant lacking L6 specificity (designated mutant X75) contained a transposed Ac element which, from experimental evidence, is putatively located in the L6 gene.

### EXAMPLE 9

### X75 CONTAINS A TRANSPOSED AC CLOSELY LINKED TO THE MUTANT L6 GENE

The L6 mutant X75 contains a transposed Ac (referred to herein as "Tr-Ac") that is not present in its resistant parent. The location of this element was mapped with respect to the L6 gene. A joint segregation analysis of Tr-Ac and the mutant L6 gene demonstrated tight linkage of these two characters. X75 was crossed to cultivar "Birio" (L6) and progeny that inherited Tr-Ac were identified by Southern analysis. Two progeny plants, D766 and D769, were test-crossed to the cultivar "Hoshangabad" (no L6). Thirty six progeny were tested for L6 rust resistance and scored for the presence f Tr-Ac. Eighteen of the progeny were susceptible and these all possessed Tr-Ac while the remaining 18 were resistant and these all lacked Tr-Ac. This is the result expected if the allele for susceptibility is an L6 gene inactivated by Tr-Ac.

The insertion site of Tr-Ac was also mapped by restriction fragment length polymorphism (RFLP) analysis in a family of 88 test-cross progeny in which L6 was segregating. A fragment of flax DNA (probe LU-1) isolated from a lambda clone containing the 3' junction between Tr-Ac and flax DNA, detected 3 RFLPs that distinguished the two parents when their DNA was cut with *Eco*RI, *Bgl*II and *Xba*I. The analysis of the segregation of these three markers and the L6 gene in the test-cross demonstrated that all four markers were closely linked. A single recombinant individual involving L6 and the RFLP marker detected in *Xba*I digested DNA was detected among the 88 progeny. As detailed below, this recombination event present in progeny plant D237 occurred within the region of the L6 gene.

### EXAMPLE 10

### RECOMBINATION IN THE VICINITY OF THE Ac INSERTION SITE ALTERS THE RESISTANCE REACTION OR SPECIFICITY OF L6

The RFLP probe LU-1 was used to establish a restriction map of the homologous regions of the resistant and susceptible parents of the test cross family and of the recombinant progeny plant D237. Three polymorphic restriction sites were detected and comparison of the maps demonstrated that a cross-over had occurred within a region of about 3kbp on either side of the Ac insertion site.

The recombinant plant D237 was selfed and 16 progeny were screened with a rust isolate that recognises L6. The progeny and were also analysed by Southern blotting to detect the recombinant chromosome. The progeny segregated for rust reaction: 6 were fully susceptible while 10 were partially resistant with restricted rust growth confined to the younger leaves. This result is consistent with the segregation of a novel gene for partial resistance which was not present in the original Forge parent. There was a complete association between the second class with novel rust reaction and the presence of the recombinant chromosome.

Thus, crossing over in the vicinity of the L6 gene results in an alteration of the L6 resistance phenotype.

### EXAMPLE 11

### INDEPENDENT L6 MUTANTS CONTAIN INSERTIONS IN THE REGION OF 0.5 TO 3.0KB OF THE Ac INSERTION SITE

Thirty four independent L6 mutants were isolated in the screen for tagged L6 genes. All but X75 were either deletions or, if not deletions, contained no transposed Ac elements (2). Analysis of mutants in the latter class using the LU-1 probe revealed that two of them (X3A and X117) contained a small (approximately 200bp) insertion in this region. In the case of X117, the mutant was compared to its resistant parent to confirm that the polymorphism was not pre-existing. In the case of X3A, the insertion and loss of L6 had occurred somatically. Mutant X3A arose as a sector. Among two shoots on a single F1 plant, one (X3A) had lost L6 and the second, X3B was wild-type for L6. DNA from the two sectors was examined using the LU-1 probe. An insertion was observed only in DNA from the mutant sector.

### EXAMPLE 12

### EXCISION OF TR-AC IN PROGENY OF X75 IS ASSOCIATED WITH REVERSION TO RUST RESISTANCE

The X75 mutant was selfed and progeny that were homozygous for the transposed Ac element were identified by Southern blotting. Selfed progeny of four such plants were in turn screened with a rust strain that recognises the L6 resistance specificity. Thirty seven resistant plants were identified among 3105 progeny. 15 of the revertants were examined by either PCR or Southern analysis for an excision fragment that would result from the excision of Ac from the L6 region. The excision marker occurred in all the plants. In two cases, the excision region was sequenced and small base changes, (an "Ac footprint") in the 8bp repeat that flanked Ac in X75 resulted from the excision process.

### EXAMPLE 13

### A DNA PROBE ADJACENT TO Ac DETECTS A SECOND LOCUS IN FLAX, CLOSELY LINKED TO THE M RESISTANCE LOCUS

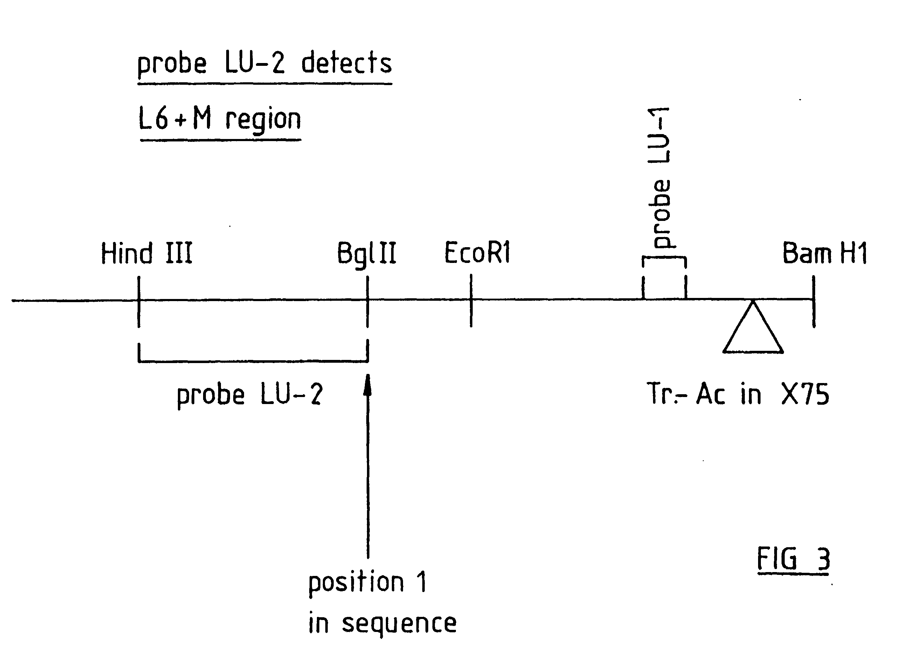

A second DNA probe, LU-2 was isolated from the same lambda clone as LU-1. These two fragments are separated by about 2kb. LU-2, like many other flax DNA probes, hybridises to two genomic fragments in most restriction digests, providing molecular evidence for the tetraploid nature of flax. In *Xba*I digests, LU-2 detects two polymorphic markers, LU-2-1 and LU-2-2 that distinguish the resistant and susceptible parents of a test-cross family of 52 progeny among which the L6 and M resistance genes segregate. Joint segregation analysis of these two RFLP markers and the L6 and M resistance genes demonstrated complete linkage of LU-2-1 with L6 and LU-2-2 with the resistance gene M. Thus, cloning the L6 locus has allowed access to the M resistance gene region. The LU-2 probe and A flanked Ac in X75 in L6 is shown in Figures 2 and 3.

### EXAMPLE 14

### RUST RESISTANCE GENES FORM A MULTIGENE FAMILY IN FLAX

The probe LU-1 and several adjacent restriction fragments from a lambda clone were used as hybridisation probes to isolate a cDNA from a library generated using leaf mRNA. A cDNA clone and several other probes from the L6 gene have been used as probes for genomic Southern analysis. These probes generally detect multiple fragments in Southern blots which indicates that flax contains a family of genes of similar sequence. All RFLPs detected with this probe map to either the L6 or M resistance gene regions.

### EXAMPLE 15

### PROBE LU-1 IDENTIFIES ALLELES OF L6

In genetic studies, the L locus of flax behaves as a single gene with multiple allelic specificities. Twelve resistance specificities map to the L locus. The probe LU-1 was used to examine a differential set of flax plants that each contain a single L allele (see Figures 2 and 3). DNA from the 12 differentials and from "Hoshangabad" which carries a "null" allele at the L locus was cut with *Xba*I, *Bgl*II and *Eco*RI. In each case, a single fragment was detected in the L differentials and each L allele could be distinguished from the null allele of "Hoshangabad". Using these three enzymes, 10 RFLP genotypes were detected. Only two pairs of L alleles, L3 and L4 and L6 and L7, could not be distinguished with this limited sample of digests.

### EXAMPLE 16

### A cDNA FROM FLAX DETECTS SEOUENCE SIMILARITY IN A WILD LINUM SPECIES

Southern analysis of DNA from the wild species, *Linum marginale* which contains genes controlling resistance against flax rust, detected strongly hybridising fragments.

### EXAMPLE 17

### DETERMINATION OF AMINO ACID SEQUENCE OF L6

The amino acid sequence of the L6 cDNA was determined and is shown in Figure 1. The amino acid sequence is shown in single letter code, single letter abbreviations for amino acids are defined below:

| Amino Acid | One-letter Symbol |
|---|---|
| Alanine | A |
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |
| Amino Acid | X |

### EXAMPLE 18

### COMPARISON OF AMINO ACID SEQUENCE OF LEUCINE RICH REPEAT FROM L6

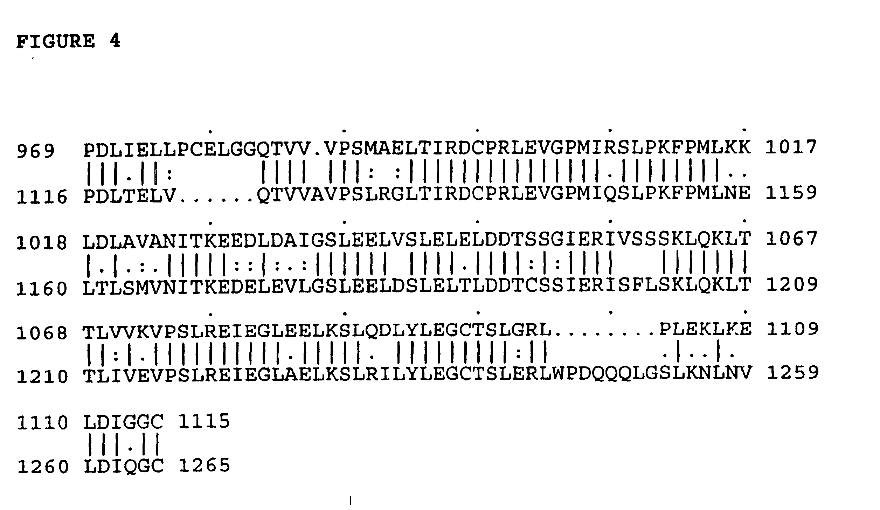

The L6 allele comprises a leucine rich repeat at amino acid residues 969 to 1115 and 1116 to 1265. This repeat was compared for identity and the comparison is shown in Figure 4. Details of the comparisons are shown below:

| | |
|---|---|
| Length | 156 amino acids |
| Gaps | 3 |
| Gap weight | 3.000 |
| Length weight | 0.100 |
| Quality | 160.4 |
| Ratio | 1.091 |
| Average match | 0.540 |
| Average mismatch | -0.396 |
| Percent similarity | 82.979% |
| Percent identity | 74.468 |

### EXAMPLE 19

### CLONING AND COMPARISON OF L LOCUS ALLELES

Using the clones L6 gene as a probe (probe LU-1), the L2 and L10 alleles were cloned on *EcoRI* DNA fragments. These alleles have different resistance specificities and control resistance to flax rust strains that carry the AL2 and AL10 avirulence genes, respectively.

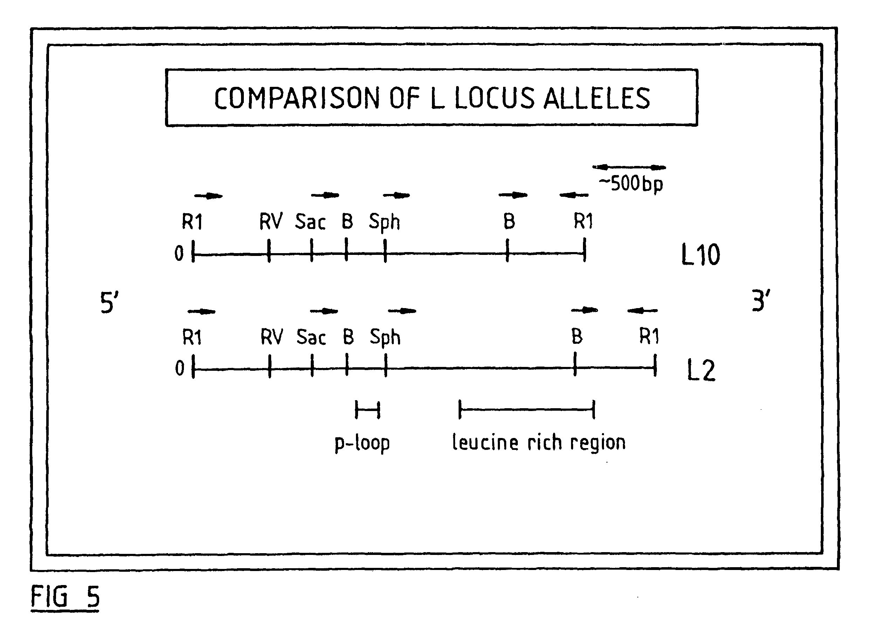

The L2 and L10 clones were subject to restriction endonuclease analysis and a schematic represented is shown in Figure 5. The L2 *EcoRI* fragment is about 400bp longer than the corresponding L10 fragment. DNA sequence analysis of both ends of the L2 and L10 fragments revealed practically identical sequence (>90%) and indicates that L2 differs from L10 by about 400bp of extra DNA. Furthermore, sequence analysis and restriction fragment mapping from internal restriction sites confirmed the extra DNA and indicated that the 5' halves of L2, L6 and L10 are very similar (>90% identical) and that the differences between the alleles occurs in the 3' half of the gene. The difference in gene length occurs in a region coding for the leucine rich repeat structure of 156 amino acids that has two copies in L6 (Figure 4) and only one copy in a cDNA from a related but non-allelic gene called FC4.

Those skilled in the art will appreciate that the invention described herein is susceptible to variations and modifications other than those specifically described.

### REFERENCES:

1. Ellis, J.G., Finnegan, E.J. and Lawrence, G.J. (1992) *Theor. Appl. Genet. 85:* 46-54.
2. Lawrence, G.J., Finnegan, E.J. and Ellis, J.G. (1993) *The Plant J. 4:* 659-669.
3. Sambrook, J., Fritsch, E.F. and Maniatis, T. (1989) *Molecular Cloning, A Laboratory Manual.* Cold Spring Harbor, NY: Cold Spring Harbor Laboratory Press.
4. Douillard and Hoffman Basic Facts about Hybridomas, in *Compendium of Immunology* Vol II, ed. by Schwartz, 1981.
5. Kohler and Milstein *Nature 256:* 495-499, 1975.
6. Kohler and Milstein *European Journal of Immunology 6:* 511-519, 1976.
7. Lawrence, G.J., Mayo, G.M.E. and Shepherd, K.W. (1991) *Phytopathology 71:* 12-19.
8. Lawrence, G.J. (1988) *Adv. Plant Pathol. 6:* 313-331.
9. Baker, B., Coupland, G., Fedoroff; N., Starlinger, P. and Schell, J. (1987) *EMBO J. 6:* 1547-1554.
10. Taylor, B.H., Finnegan, E.J., Dennis, E.S. and Peacock, W.J. (1989) *Plant Mol. Biol. 13:* 109-118.
11. Finnegan, E.J., Lawrence, GJ., Dennis, E.S. and Ellis, J.G. (1993) *Plant. Mol. Biol.* in press.
12. Ellis, J.G., Llewellyn, D.J., Walker, J.C., Dennis, E.S. and Peacock, W.J. (1987) *EMBO J. 6:* 3203-3208.
13. Lawrence, G.J., Ellis, J.G., Finnegan, E.J., Dennis, E.S. and Peacock, W.J. (1989) Tagging rust resistance genes in flax. In Breeding Research: The Key to the Survival of the Earth (Iyama, S. and Takeda, G, eds). Tsukuba: The Organising Committee, The 7th Int. Congr. of SABRAO, pp. 535-538.
14. Traut (1994) *Eur. J. Biochem. 222.- 9-12.*
15. Miklas *et al* (1993) Theor Appl Genet 85: 745-749

### SEQUENCE LISTING

(1) GENERAL INFORMATION:
   (i) APPLICANT: COMMONWEALTH SCIENTIFIC AND INDUSTRIAL RESEARCH ORGANISATION
   (ii) TITLE OF INVENTION: GENETIC SEQUENCES CONFERRING DISEASE RESISTANCE IN PLANTS AND USES THEREFOR
   (iii) NUMBER OF SEQUENCES: 13
   (iv) CORRESPONDENCE ADDRESS:
      (A) ADDRESSEE: DAVIES COLLISON CAVE
      (B) STREET: 1 LITTLE COLLINS STREET
      (C) CITY: MELBOURNE
      (D) STATE: VICTORIA
      (E) COUNTRY: AUSTRALIA
      (F) ZIP: 3000
   (v) COMPUTER READABLE FORM:
      (A) MEDIUM TYPE: Floppy disk
      (B) COMPUTER: IBM PC compatible
      (C) OPERATING SYSTEM: PC=DOS/MS-DOS
      (D) SOFTWARE: Patentln Release #1.0, Version #1.25
   (vi) CURRENT APPLICATION DATA:
      (A) APPLICATION NUMBER: PCT INTERNATIONAL
      (B) FILING DATE: 21-APR-1995
   (vii) PRIOR APPLICATION DATA:
      (A) APPLICATION NUMBER: PM5231/94 (AU)
      (B) FILING DATE: 21-APR-1994
      (A) APPLICATION NUMBER: PM8103/94 (AU)
      (B) FILING DATE: 14-SEP-1994
   (viii) ATTORNEY/AGENT INFORMATION:
      (A) NAME: HUGHES DR, E JOHN L
      (C) REFERENCE/DOCKET NUMBER: EJH/EK
   (ix) TELECOMMUNICATION INFORMATION:
      (A) TELEPHONE: +61 3 254 2777
      (B) TELEFAX: +61 3 254 2770
(2) INFORMATION FOR SEQ ID NO:1:
   (i) SEQUENCE CHARACTERISTICS:
      (A) LENGTH: 4841 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 163..789
   (ix) FEATURE:
      (A) NAME/KEY: CDS
      (B) LOCATION: 1097..2203
   (ix) FEATURE:
      (A) NAME/KEY: CDS
      (B) LOCATION: 2669..4525
   (ix) FEATURE:
      (A) NAME/KEY: CDS
      (B) LOCATION: 2293..2586
   (xi) SEQUENCE DESCRIPTION: SEQ ID NO:1:
   GAGCTCAGAA GTAAGGGATA AGGCAAGAAG CAGAGGAGCA GAGAGAAGAA CCAGCAAAGC      60
   ACATGCAAAT TGAAGCAGGC AAGAACAGTT ACTGGAAATT CATTTATCTC TGCTTTCAAT     120
   TTCTATCCTT CAGATCATTT CTGCTCAATT GAATCACTAG TC ATG AGT TAT TTG       174
                                                  Met Ser Tyr Leu
                                                    1
   AGA GAA GTT GCT ACT GCT GTT GCC TTG CTT CTC CCT TTC ATT CTT CTC       222
   Arg Glu Val Ala Thr Ala Val Ala Leu Leu Leu Pro Phe Ile Leu Leu
     5                  10                  15                  20
   AAC AAG TTT TGG AGA CCA AAT TCC AAA GAC TCA ATC GTC AAC GAT GAT       270
   Asn Lys Phe Trp Arg Pro Asn Ser Lys Asp Ser Ile Val Asn Asp Asp
                    25                  30                  35
   GAC GAT TCA ACA TCT GAA GTT GAT GCC ATA TCC GAC TCC ACA AAT CCC       318
   Asp Asp Ser Thr Ser Glu Val Asp Ala Ile Ser Asp Ser Thr Asn Pro
                40                  45                  50
   TCT GGT TCA TTT CCC TCC GTG GAG TAT GAA GTG TTT TTG AGT TTC AGG       366
   Ser Gly Ser Phe Pro Ser Val Glu Tyr Glu Val Phe Leu Ser Phe Arg
            55                  60                  65
   GGT CCA GAT ACT CGT GAA CAG TTC ACC GAT TTC CTA TAT CAG TCT CTC       414
   Gly Pro Asp Thr Arg Glu Gln Phe Thr Asp Phe Leu Tyr Gln Ser Leu
        70                  75                  80
   CGT CGC TAT AAG ATT CAC ACT TTT AGG GAC GAC GAT GAG CTA CTC AAA        462
   Arg Arg Tyr Lys Ile His Thr Phe Arg Asp Asp Asp Glu Leu Leu Lys
    85                 90                  95                 100
   GGA AAA GAA ATA GGG CCC AAC CTC CTA CGA GCA ATT GAT CAG TCC AAA        510
   Gly Lys Glu Ile Gly Pro Asn Leu Leu Arg Ala Ile Asp Gln Ser Lys
                   105                 110                 115
   ATT TAC GTC CCG ATC ATA TCG AGC GGA TAT GCT GAT AGT AAG TGG TGT        558
   Ile Tyr Val Pro Ile Ile Ser Ser Gly Tyr Ala Asp Ser Lys Trp Cys
               120                 125                 130
   CTT ATG GAG CTT GCT GAA ATT GTG AGA CGT CAA GAG GAG GAC CCT CGA        606
   Leu Met Glu Leu Ala Glu Ile Val Arg Arg Gln Glu Glu Asp Pro Arg
           135                 140                 145
   CGC ATC ATA CTT CCT ATT TTT TAT ATG GTG GAT CCA AGT GAC GTA CGA        654
   Arg Ile Ile Leu Pro Ile Phe Tyr Met Val Asp Pro Ser Asp Val Arg
       150                 155                 160
   CAT CAG ACT GGA TGT TAT AAA AAA GCA TTT CGA AAA CAC GCA AAT AAA        702
   His Gln Thr Gly Cys Tyr Lys Lys Ala Phe Arg Lys His Ala Asn Lys
   165                 170                 175                 180
   TTT GAT GGA CAG ACC ATA CAA AAC TGG AAA GAT GCT CTA AAG AAG GTC        750
   Phe Asp Gly Gln Thr Ile Gln Asn Trp Lys Asp Ala Leu Lys Lys Val
                   185                 190                 195
   GGA GAC TTA AAA GGA TGG CAC ATC GGA AAG AAT GAC AAG TATGTAATCC        799
   Gly Asp Leu Lys Gly Trp His Ile Gly Lys Asn Asp Lys
               200                 205
   TCATCCTAGC CTTTTATCAT TCAGTACAAC TTATTTGTTT TGTCTTGCAT ATACTTACTT     859
   GTTTATTGAC TAACTTTCAA ATTGCATATT AACATTCTAG GAAGATTTAA ATTGGACTCG     919
   GGTCACAACT GAACCATATT ATTTTACACG ACAAAGCACG AGCTATTCCT AATGTAATTA     979
   AAGTTAAAAT GCTCAATAAC GTGGAATATA TACTTGTATA CAGGGGCAAC AAACTATAAA    1039
   TTGAAATATT ATTTCATATG AAGTTAACAT CTCAAAAATG TATTTAATAC TTGTAGG       1096
   CAG GGA GCT ATA GCA GAC AAA GTA TCA GCA GAT ATA TGG TCA CAC ATA       1144
   Gln Gly Ala Ile Ala Asp Lys Val Ser Ala Asp Ile Trp Ser His Ile
     1               5                  10                  15
   AGC AAG GAA AAT CTC ATT TTA GAA ACC GAT GAG TTG GTT GGA ATT GAT       1192
   Ser Lys Glu Asn Leu Ile Leu Glu Thr Asp Glu Leu Val Gly Ile Asp
                   20                  25                  30
   GAT CAC ATA ACA GCC GTA TTA GAA AAA TTG AGT TTA GAC TCT GAA AAT       1240
   Asp His Ile Thr Ala Val Leu Glu Lys Leu Ser Leu Asp Ser Glu Asn
               35                  40                  45
   GTG ACA ATG GTC GGC CTT TAT GGT ATG GGT GGA ATA GGC AAG ACG ACC        1288
   Val Thr Met Val Gly Leu Tyr Gly Met Gly Gly Ile Gly Lys Thr Thr
        50                  55                  60
   ACT GCA AAG GCG GTT TAT AAC AAG ATT TCT TCT TGT TTC GAT TGT TGT        1336
   Thr Ala Lys Ala Val Tyr Asn Lys Ile Ser Ser Cys Phe Asp Cys Cys
    65                  70                  75                  80
   TGT TTT ATT GAC AAC ATA CGT GAA ACA CAA GAG AAG GAT GGC GTT GTT        1384
   Cys Phe Ile Asp Asn Ile Arg Glu Thr Gln Glu Lys Asp Gly Val Val
                    85                  90                  95
   GTT TTG CAA AAG AAG CTA GTA TCT GAA ATT TTG AGG ATC GAT TCG GGT        1432
   Val Leu Gln Lys Lys Leu Val Ser Glu Ile Leu Arg Ile Asp Ser Gly
               100                 105                 110
   TCG GTT GGA TTT AAT AAT GAT AGT GGC GGA CGG AAG ACG ATA AAG GAG        1480
   Ser Val Gly Phe Asn Asn Asp Ser Gly Gly Arg Lys Thr Ile Lys Glu
           115                   120                 125
   AGA GTT TCG AGG TTC AAA ATT CTT GTC GTT CTC GAT GAT GTG GAT GAG        1528
   Arg Val Ser Arg Phe Lys Ile Leu Val Val Leu Asp Asp Val Asp Glu
       130                 135                 140
   AAG TTT AAA TTT GAA GAT ATG TTG GGA AGT CCT AAA GAT TTT ATT TCT        1576
   Lys Phe Lys Phe Glu Asp Met Leu Gly Ser Pro Lys Asp Phe Ile Ser
   145                 150                 155                 160
   CAA AGT AGA TTC ATT ATT ACT TCA AGA AGT ATG AGA GTT TTG GGT ACT        1624
   Gln Ser Arg Phe Ile Ile Thr Ser Arg Ser Met Arg Val Leu Gly Thr
                   165                 170                 175
   TTG AAT GAG AAT CAA TGC AAG TTG TAT GAA GTT GGA TCG ATG AGC AAA        1672
   Leu Asn Glu Asn Gln Cys Lys Leu Tyr Glu Val Gly Ser Met Ser Lys
               180                 185                 190
   CCA CGT TCG CTT GAA CTC TTC TCC AAG CAT GCA TTC AAA AAG AAT ACG        1720
   Pro Arg Ser Leu Glu Leu Phe Ser Lys His Ala Phe Lys Lys Asn Thr
           195                 200                 205
   CCT CCA TCG TAT TAT GAG ACT CTA GCA AAT GAC GTC GTA GAT ACT ACA        1768
   Pro Pro Ser Tyr Tyr Glu Thr Leu Ala Asn Asp Val Val Asp Thr Thr
       210                 215                 220
   GCA GGA CTT CCA TTG ACT CTG AAG GTT ATA GGA TCG CTT TTA TTT AAA        1816
   Ala Gly Leu Pro Leu Thr Leu Lys Val Ile Gly Ser Leu Leu Phe Lys
   225                 230                 235                 240
   CAA GAG ATT GCG GTT TGG GAA GAC ACG TTG GAA CAA TTA CGT AGA ACA        1864
   Gln Glu Ile Ala Val Trp Glu Asp Thr Leu Glu Gln Leu Arg Arg Thr
                   245                 250                 255
   CTT AAC CTT GAT GAG GTT TAT GAT AGG CTA AAA ATA AGT TAT GAT GCG        1912
   Leu Asn Leu Asp Glu Val Tyr Asp Arg Leu Lys Ile Ser Tyr Asp Ala
               260                 265                 270
   TTG AAC CCG GAG GCA AAA GAG ATT TTC TTG GAT ATA GCT TGC TTC TTC        1960
   Leu Asn Pro Glu Ala Lys Glu Ile Phe Leu Asp Ile Ala Cys Phe Phe
           275                 280                 285
   ATC GGA CAA AAT AAA GAA GAA CCG TAT TAC ATG TGG ACC GAC TGT AAT        2008
   Ile Gly Gln Asn Lys Glu Glu Pro Tyr Tyr Met Trp Thr Asp Cys Asn
       290                 295                 300
   TTT TAT CCA GCA AGT AAT ATT ATT TTT CTC ATT CAA AGA TGT ATG ATA        2056
   Phe Tyr Pro Ala Ser Asn Ile Ile Phe Leu Ile Gln Arg Cys Met Ile
   305                 310                 315                 320
   CAA GTT GGG GAT GAT GAT GAG TTT AAA ATG CAC GAC CAA CTT AGA GAT        2104
   Gln Val Gly Asp Asp Asp Glu Phe Lys Met His Asp Gln Leu Arg Asp
                   325                 330                 335
   ATG GGT AGA GAA ATT GTG AGA CGA GAG GAT GTA CTG CCG TGG AAG AGA        2152
   Met Gly Arg Glu Ile Val Arg Arg Glu Asp Val Leu Pro Trp Lys Arg
               340                 345                 350
   AGT AGA ATA TGG TCG GCA GAA GAA GGG ATT GAT CTC TTG CTG AAC AAA        2200
   Ser Arg Ile Trp Ser Ala Glu Glu Gly Ile Asp Leu Leu Leu Asn Lys
           355                 360                 365
   AAG GTATTCAGTT TTATTTAAAA TTAAATATTC ATATATTATT ACACATACTT             2253
   Lys
   TAAATCACAT AACTCATTAC GTTCCTTCTC CAATTACAG GGA TCA AGT AAA GTA         2307
                                              Gly Ser Ser Lys Val
                                                1               5
   AAA GCA ATT AGC ATA CCC TGG GGT GTC AAG TAT GAG TTT AAG AGC GAA        2355
   Lys Ala Ile Ser Ile Pro Trp Gly Val Lys Tyr Glu Phe Lys Ser Glu
                   10                  15                  20
   TGT TTC TTG AAT TTG TCA GAG TTG AGA TAC CTC CAT GCA AGG GAA GCC        2403
   Cys Phe Leu Asn Leu Ser Glu Leu Arg Tyr Leu His Ala Arg Glu Ala
               25                  30                  35
   ATG CTT ACC GGA GAT TTC AAC AAT CTT CTC CCG AAT TTA AAG TGG CTT        2451
   Met Leu Thr Gly Asp Phe Asn Asn Leu Leu Pro Asn Leu Lys Trp Leu
           40                  45                  50
   GAG TTG CCA TTT TAC AAA CAT GGA GAG GAT GAT CCT CCT TTG ACC AAT        2499
   Glu Leu Pro Phe Tyr Lys His Gly Glu Asp Asp Pro Pro Leu Thr Asn
       55                  60                  65
   TAT ACC ATG AAA AAT CTG ATA ATT GTT ATT CTT GAG CAT AGC CAC ATA        2547
   Tyr Thr Met Lys Asn Leu Ile Ile Val Ile Leu Glu His Ser His Ile
   70                  75                  80                  85
   ACG GCT GAT GAT TGG GGA GGT TGG AGG CAT ATG ATG AAG GTGTGTTGTT         2596
   Thr Ala Asp Asp Trp Gly Gly Trp Arg His Met Met Lys
                   90                  95
   TTTCAGCTGT TCATATGAAG GTGTGTTATC TTCTTATTTG TTCTTCATAT TTCTGTTTTA    2656
   ATCTGCTGTC AG ATG GCT GAG AGG CTG AAA GTT GTA CGA CTT GCT TCA        2704
                 Met Ala Glu Arg Leu Lys Val Val Arg Leu Ala Ser
                   1               5                  10
   AAC TAT AGT TTG TAC GGA AGA CGT GTT CGC CTT TCT GAC TGT TGG CGC      2752
   Asn Tyr Ser Leu Tyr Gly Arg Arg Val Arg Leu Ser Asp Cys Trp Arg
           15                  20                  25
   TTC CCC AAA AGC ATT GAG GTA TTA TCC ATG ACT GCG ATA GAA ATG GAT      2800
   Phe Pro Lys Ser Ile Glu Val Leu Ser Met Thr Ala Ile Glu Met Asp
       30                  35                  40
   GAA GTT GAT ATT GGG GAG TTA AAG AAG CTA AAG ACG TTG GTT CTG AAA      2848
   Glu Val Asp Ile Gly Glu Leu Lys Lys Leu Lys Thr Leu Val Leu Lys
    45                  50                  55                  60
   TTC TGT CCA ATA CAA AAG ATA AGT GGG GGA ACC TTT GGT ATG TTG AAG      2896
   Phe Cys Pro Ile Gln Lys Ile Ser Gly Gly Thr Phe Gly Met Leu Lys
                   65                  70                  75
   GGA CTT CGA GAG CTT TGT CTC GAA TTC AAC TGG GGG ACA AAT TTG AGA      2944
   Gly Leu Arg Glu Leu Cys Leu Glu Phe Asn Trp Gly Thr Asn Leu Arg
               80                  85                  90
   GAG GTA GTT GCC GAT ATT GGT CAA CTT TCA TCT CTC AAA GTC TTG AAA      2992
   Glu Val Val Ala Asp Ile Gly Gln Leu Ser Ser Leu Lys Val Leu Lys
           95                  100                 105
   ACA ACC GGA GCT AAG GAG GTC GAG ATT AAT GAA TTT CCA TTA GGT TTG      3040
   Thr Thr Gly Ala Lys Glu Val Glu Ile Asn Glu Phe Pro Leu Gly Leu
       110                 115                 120
   AAG GAG TTA TCC ACT TCA TCT CGG ATT CCG AAT CTT TCA CAG TTG TTG      3088
   Lys Glu Leu Ser Thr Ser Ser Arg Ile Pro Asn Leu Ser Gln Leu Leu
   125                 130                 135                 140
   GAT TTG GAG GTA CTG AAG GTT TAT GAT TGC AAG GAT GGA TTT GAC ATG      3136
   Asp Leu Glu Val Leu Lys Val Tyr Asp Cys Lys Asp Gly Phe Asp Met
                   145                 150                 155
   CCT CCT GCT AGT CCG AGT GAA GAT GAA AGT AGT GTG TGG TGG AAG GTA      3184
   Pro Pro Ala Ser Pro Ser Glu Asp Glu Ser Ser Val Trp Trp Lys Val
               160                 165                 170
   TCC AAG TTG AAG TCT TTG CAA CTC GAG AAG ACA AGA ATC AAT GTC AAC      3232
   Ser Lys Leu Lys Ser Leu Gln Leu Glu Lys Thr Arg Ile Asn Val Asn
           175                 180                 185
   GTT GTG GAT GAT GCT TCT TCC GGT GGT CAC CTC CCT CGT TAC TTA CTA      3280
   Val Val Asp Asp Ala Ser Ser Gly Gly His Leu Pro Arg Tyr Leu Leu
       190                 195                 200
   CCA ACA TCC CTA ACC TAT CTT AAA ATT TAT CAG TGT ACA GAA CCA ACG        3328
   Pro Thr Ser Leu Thr Tyr Leu Lys Ile Tyr Gln Cys Thr Glu Pro Thr
   205                 210                 215                 220
   TGG CTT CCA GGA ATA GAG AAC TTG GAG AAT TTG ACT TCG CTG GAA GTC        3376
   Trp Leu Pro Gly Ile Glu Asn Leu Glu Asn Leu Thr Ser Leu Glu Val
                   225                 230                 235
   AAC GAC ATC TTC CAA ACT CTT GGA GGT GAC TTG GAT GGG CTA CAA GGG        3424
   Asn Asp Ile Phe Gln Thr Leu Gly Gly Asp Leu Asp Gly Leu Gln Gly
               240                 245                 250
   TTG AGA TCA TTG GAA ATT CTT AGG ATT CGG AAA GTA AAT GGT TTA GCT        3472
   Leu Arg Ser Leu Glu Ile Leu Arg Ile Arg Lys Val Asn Gly Leu Ala
           255                 260                 265
   CGG ATC AAA GGG CTT AAG GAT CTC TTG TGT TCT TCT ACC TGC AAG TTG        3520
   Arg Ile Lys Gly Leu Lys Asp Leu Leu Cys Ser Ser Thr Cys Lys Leu
       270                 275                 280
   CGG AAA TTT TAT ATT ACA GAA TGC CCC GAC CTC ATT GAG TTA CTC CCA        3568
   Arg Lys Phe Tyr Ile Thr Glu Cys Pro Asp Leu Ile Glu Leu Leu Pro
   285                 290                 295                 300
   TGC GAA CTC GGC GGC CAA ACA GTA GTA GTC CCC TCT ATG GCA GAA CTG        3616
   Cys Glu Leu Gly Gly Gln Thr Val Val Val Pro Ser Met Ala Glu Leu
                   305                 310                 315
   ACC ATT AGG GAT TGT CCA CGG CTG GAG GTT GGC CCG ATG ATA AGA TCA        3664
   Thr Ile Arg Asp Cys Pro Arg Leu Glu Val Gly Pro Met Ile Arg Ser
               320                 325                 330
   CTC CCA AAG TTC CCA ATG CTA AAG AAG TTG GAC CTC GCG GTG GCA AAT        3712
   Leu Pro Lys Phe Pro Met Leu Lys Lys Leu Asp Leu Ala Val Ala Asn
           335                 340                 345
   ATA ACT AAA GAG GAG GAT CTG GAT GCG ATT GGA TCC CTA GAA GAG TTG        3760
   Ile Thr Lys Glu Glu Asp Leu Asp Ala Ile Gly Ser Leu Glu Glu Leu
       350                 355                 360
   GTT AGT TTG GAG TTA GAG TTA GAC GAT ACA TCT TCC GGT ATA GAG AGG        3808
   Val Ser Leu Glu Leu Glu Leu Asp Asp Thr Ser Ser Gly Ile Glu Arg
   365                 370                 375                 380
   ATA GTT TCC TCT TCG AAG CTG CAA AAG TTA ACT ACA CTC GTA GTG AAG        3856
   Ile Val Ser Ser Ser Lys Leu Gln Lys Leu Thr Thr Leu Val Val Lys
                   385                 390                 395
   GTG CCG AGT TTG CGG GAG ATT GAA GGG CTT GAA GAG TTG AAG TCT TTA        3904
   Val Pro Ser Leu Arg Glu Ile Glu Gly Leu Glu Glu Leu Lys Ser Leu
               400                 405                 410
   CAA GAT TTG TAT CTA GAG GGT TGC ACG TCG TTG GGG AGA CTA CCA CTG        3952
   Gln Asp Leu Tyr Leu Glu Gly Cys Thr Ser Leu Gly Arg Leu Pro Leu
           415                 420                 425
   GAG AAG CTG AAG GAG CTA GAC ATT GGA GGA TGC CCT GAC CTC ACT GAG      4000
   Glu Lys Leu Lys Glu Leu Asp Ile Gly Gly Cys Pro Asp Leu Thr Glu
       430                 435                 440
   TTA GTC CAA ACA GTA GTA GCA GTC CCC TCT TTG AGA GGA CTG ACC ATT      4048
   Leu Val Gln Thr Val Val Ala Val Pro Ser Leu Arg Gly Leu Thr Ile
   445                 450                 455                 460
   AGG GAT TGT CCA CGG CTG GAG GTT GGT CCA ATG ATA CAA TCT CTT CCA      4096
   Arg Asp Cys Pro Arg Leu Glu Val Gly Pro Met Ile Gln Ser Leu Pro
                   465                 470                 475
   AAG TTC CCA ATG CTA AAT GAA TTG ACG CTC TCG ATG GTA AAT ATC ACT      4144
   Lys Phe Pro Met Leu Asn Glu Leu Thr Leu Ser Met Val Asn Ile Thr
               480                 485                 490
   AAG GAG GAT GAG CTG GAG GTG CTT GGA TCC CTA GAA GAG TTG GAT AGT      4192
   Lys Glu Asp Glu Leu Glu Val Leu Gly Ser Leu Glu Glu Leu Asp Ser
           495                 500                 505
   TTG GAG TTA ACG TTA GAC GAT ACA TGT TCC AGC ATA GAG AGG ATA TCT      4240
   Leu Glu Leu Thr Leu Asp Asp Thr Cys Ser Ser Ile Glu Arg Ile Ser
       510                 515                 520
   TTC TTG TCG AAG CTG CAA AAG TTA ACT ACA CTC ATA GTG GAG GTG CCG      4288
   Phe Leu Ser Lys Leu Gln Lys Leu Thr Thr Leu Ile Val Glu Val Pro
   525                 530                 535                 540
   AGT TTG CGG GAG ATT GAA GGT CTT GCA GAG TTG AAG TCT TTA CGA ATT      4336
   Ser Leu Arg Glu Ile Glu Gly Leu Ala Glu Leu Lys Ser Leu Arg Ile
                   545                 550                 555
   TTG TAT CTA GAA GGA TGC ACG TCG TTG GAA AGA CTG TGG CCT GAT CAA      4384
   Leu Tyr Leu Glu Gly Cys Thr Ser Leu Glu Arg Leu Trp Pro Asp Gln
               560                 565                 570
   CAA CAG TTG GGT AGT CTG AAG AAC CTG AAT GTG CTC GAC ATC CAA GGT      4432
   Gln Gln Leu Gly Ser Leu Lys Asn Leu Asn Val Leu Asp Ile Gln Gly
           575                 580                 585
   TGT AAA AGC TTG AGT GTT GAC CAT CTC TCT GCA CTC AAG ACC ACT CTA      4480
   Cys Lys Ser Leu Ser Val Asp His Leu Ser Ala Leu Lys Thr Thr Leu
       590                 595                 600
   CCG CCC AGG GCG AGG ATA ACA TGG CCC GAT CAG CCC TAC AGA TGACGGTAGG   4532
   Pro Pro Arg Ala Arg Ile Thr Trp Pro Asp Gln Pro Tyr Arg
   605                 610                 615
   AATTAATGCA AGTGACAGTG ATGACATAGT TGTGATGGCT TGCACCTGAT CAACCTTGTT    4592
   CTTCATGGGG TTTTCTCCCC AGTGAGATCT TAATATCCAA AATTCTGGTT TGTTCAGAGG    4652
   TTATATGGTT CAGTTTTTCA CCATAATATT TTCTACGGCA TAGCGCAAAC TACTTCTAGT    4712
   ATATAGATAT AGGCAATATA TATAACATCC AACTCTGTTT TACTCACTCC TCTCATCTTC    4772
   TCACTCGATT ATGTTCCATT TCTAAAATCC ATTATTCATC GCCTTATATT CATAATTATG 4832
   TAATTATTT 4841
(2) INFORMATION FOR SEO ID NO:2:
   (i) SEQUENCE CHARACTERISTICS:
      (A) LENGTH: 208 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 369 amino acids
      (B) TYPE: amino acid
      (D) TOPOLOGY: linear
   (ii) MOLECULE TYPE: protein
   (xi) SEQUENCE DESCRIPTION: SEQ ID NO:3:
   Gln Gly Ala Ile Ala Asp Lys Val Ser Ala Asp Ile Trp Ser His Ile
    1               5                  10                  15
   Ser Lys Glu Asn Leu Ile Leu Glu Thr Asp Glu Leu Val Gly Ile Asp
               20                  25                  30
   Asp His Ile Thr Ala Val Leu Glu Lys Leu Ser Leu Asp Ser Glu Asn
           35                  40                  45
   Val Thr Met Val Gly Leu Tyr Gly Met Gly Gly Ile Gly Lys Thr Thr
       50                  55                  60
   Thr Ala Lys Ala Val Tyr Asn Lys Ile Ser Ser Cys Phe Asp Cys Cys
   65                  70                  75                  80
   Cys Phe Ile Asp Asn Ile Arg Glu Thr Gln Glu Lys Asp Gly Val Val
                   85                  90                  95
   Val Leu Gln Lys Lys Leu Val Ser Glu Ile Leu Arg Ile Asp Ser Gly
               100                 105                 110
   Ser Val Gly Phe Asn Asn Asp Ser Gly Gly Arg Lys Thr Ile Lys Glu
           115                 120                 125
   Arg Val Ser Arg Phe Lys Ile Leu Val Val Leu Asp Asp Val Asp Glu
       130                 135                 140
   Lys Phe Lys Phe Glu Asp Met Leu Gly Ser Pro Lys Asp Phe Ile Ser
   145                 150                 155                 160
   Gln Ser Arg Phe Ile Ile Thr Ser Arg Ser Met Arg Val Leu Gly Thr
                   165                 170                 175
   Leu Asn Glu Asn Gln Cys Lys Leu Tyr Glu Val Gly Ser Met Ser Lys
               180                 185                 190
   Pro Arg Ser Leu Glu Leu Phe Ser Lys His Ala Phe Lys Lys Asn Thr
           195                 200                 205
   Pro Pro Ser Tyr Tyr Glu Thr Leu Ala Asn Asp Val Val Asp Thr Thr
       210                 215                 220
   Ala Gly Leu Pro Leu Thr Leu Lys Val Ile Gly Ser Leu Leu Phe Lys
   225                 230                 235                 240
   Gln Glu Ile Ala Val Trp Glu Asp Thr Leu Glu Gln Leu Arg Arg Thr
                   245                 250                 255
   Leu Asn Leu Asp Glu Val Tyr Asp Arg Leu Lys Ile Ser Tyr Asp Ala
               260                 265                 270
   Leu Asn Pro Glu Ala Lys Glu Ile Phe Leu Asp Ile Ala Cys Phe Phe
           275                 280                 285
   Ile Gly Gln Asn Lys Glu Glu Pro Tyr Tyr Met Trp Thr Asp Cys Asn
       290                 295                 300
   Phe Tyr Pro Ala Ser Asn Ile Ile Phe Leu Ile Gln Arg Cys Met Ile
   305                 310                 315                 320
   Gln Val Gly Asp Asp Asp Glu Phe Lys Met His Asp Gln Leu Arg Asp
                   325                 330                 335
   Met Gly Arg Glu Ile Val Arg Arg Glu Asp Val Leu Pro Trp Lys Arg
               340                 345                 350
   Ser Arg Ile Trp Ser Ala Glu Glu Gly Ile Asp Leu Leu Leu Asn Lys
           355                 360                 365
   Lys
(2) INFORMATION FOR SEQ ID NO:4:
   (i) SEQUENCE CHARACTERISTICS:
      (A) LENGTH: 98 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Gly Ser Ser Lys Val Lys Ala Ile Ser Ile Pro Trp Gly Val Lys Tyr
   1               5                   10                  15
   Glu Phe Lys Ser Glu Cys Phe Leu Asn Leu Ser Glu Leu Arg Tyr Leu
               20                  25                  30
   His Ala Arg Glu Ala Met Leu Thr Gly Asp Phe Asn Asn Leu Leu Pro
   35 40 45
   Asn Leu Lys Trp Leu Glu Leu Pro Phe Tyr Lys His Gly Glu Asp Asp
   50 55 60
   Pro Pro Leu Thr Asn Tyr Thr Met Lys Asn Leu Ile Ile Val Ile Leu
   65 70 75 80
   Glu His Ser His Ile Thr Ala Asp Asp Trp Gly Gly Trp Arg His Met
   85 90 95
   Met Lys
(2) INFORMATION FOR SEQ ID NO:5:
   (i) SEQUENCE CHARACTERISTICS:
      (A) LENGTH: 619 amino acids
      (B) TYPE: amino acid
      (D) TOPOLOGY: linear
   (ii) MOLECULE TYPE: protein
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..90
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 29 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Val Cys Cys Phe Ser Ala Val His Met Lys Val Cys Tyr Leu Leu Ile
     1               5                  10                  15
   Cys Ser Ser Tyr Phe Cys Phe Asn Leu Leu Ser Asp Gly
                   20                  25
(2) INFORMATION FOR SEQ ID NO:8:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:8:
   (xi(i)) /: Alternative amino acid
(2) INFORMATION FOR SEQ ID NO:9:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 9:
   Gly Met Gly Gly Ile Gly Lys Thr Thr
   1               5
(2) INFORMATION FOR SEQ ID NO:10:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:10:
   Gly Leu Tyr Gly Met Gly Gly Ile Gly Lys Thr Thr
   1               5                   10
(2) INFORMATION FOR SEQ ID NO:11:
   (i) SEQUENCE CHARACTERISTICS:
      (A) LENGTH: 840 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:11:
   AAAAAAAAAT TATAAGTTAA CAATTATACG TATGTTCCTA ACAATTGCTA AATAAATATT 60
   ATCTCATCCT TCACTCCATT TTAGCAATTG TTAAAATTTT GGAATCGATA AATCTCGATC 120
   CACTAACCAC TTCAAAATAG AAAAATAATG CGTAGCACAC AAACAACAGG ATTGGTGGCC 180
   CCTTGATCAT GTAGCCAATT AGGTGATTCT TGTGGACAAG TAGAACTAGT TGTACTCGCT 240
   AACCGGTCCG ATTTGAAATT TGACCCAGGT TTCGAAATTC TAACCAACCC AAATGGTTGA 300
   CTGACTTGTG GGCCTGCATT ATTTGTAGAT AAGAAAGAGT TTACAAATGG TTGGCTTACA 360
   TTGAATTTGT GGAGCAATAA TTGAGTTTTT GAATGGAGGA TACTGAAAGC CAGTGGGAGC 420
   TCAGAAGTAA GGGATAAGGC AAGAAGCAGA GGAGCAGAGA GAAGAACCAG CAAAGCACAT 480
   GCAAATTGAA GCAGGCAAGA ACAGTTACTG GAAATTCATT TATCTCTGCT TTCAATTTCT 540
   ATCCTTCAGA TCATTTCTGC TCAATTGAAT CACTAGTCAT GAGTTATTTG AGAGAAGTTG 600
   CTACTGCTGT TGCCTTGCTT CTCCCTTTCA TTCTTCTCAA CAAGTTTTGG AGACCAAATT 660
   CCAAAGACTC AATCGTCAAC GATGATGACG ATTCAACATC TGAAGTTGAT GCCATATCCG 720
   ACTCCACAAA TCCCTCTGGT TCATTTCCCT CCGTGGAGTA TGAAGTGTTT TTGAGTTTCA 780
   GGGGTCCAGA TACTCGTGAA CAGTTCACCG ATTTCCTATA TCAGTCTCTC CGTCGCTATA 840
(2) INFORMATION FOR SEQ ID NO:12:
   (i) SEQUENCE CHARACTERISTICS:
      (A) LENGTH: 150 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:12:
   Pro Asp Leu Thr Glu Leu Val Gln Thr Val Val Ala Val Pro Ser Leu
   1 5 10 15
   Arg Gly Leu Thr Ile Arg Asp Cys Pro Arg Leu Glu Val Gly Pro Met
   20 25 30
   Ile Gln Ser Leu Pro Lys Phe Pro Met Leu Asn Glu Leu Thr Leu Ser
   35 40 45
   Met Val Asn Ile Thr Lys Glu Asp Glu Leu Glu Val Leu Gly Ser Leu
   50 55 60
   Glu Glu Leu Asp Ser Leu Glu Leu Thr Leu Asp Asp Thr Cys Ser Ser
   65 70 75 80
   Ile Glu Arg Ile Ser Phe Leu Ser Lys Leu Gln Lys Leu Thr Thr Leu
   85 90 95
   Ile Val Glu Val Pro Ser Leu Arg Glu Ile Glu Gly Leu Ala Glu Leu
   100 105 110
   Lys Ser Leu Arg Ile Leu Tyr Leu Glu Gly Cys Thr Ser Leu Glu Arg
   115 120 125
   Leu Trp Pro Asp Gln Gln Gln Leu Gly Ser Leu Lys Asn Leu Asn Val
   130 135 140
   Leu Asp Ile Gln Gly Cys
   145 150
(2) INFORMATION FOR SEQ ID NO:13:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:13:
   Ile Leu Val Val Leu Asp Asp Val Asp
   1 5
(2) INFORMATION FOR SEQ ID NO:14:
   (i) SEQUENCE CHARACTERISTICS:
      (A) LENGTH: 147 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:14:
   Pro Asp Leu Ile Glu Leu Leu Pro Cys Glu Leu Gly Gly Gln Thr Val
   1               5                   10                  15
   Val Val Pro Ser Met Ala Glu Leu Thr Ile Arg Asp Cys Pro Arg Leu
               20                  25                  30
   Glu Val Gly Pro Met Ile Arg Ser Leu Pro Lys Phe Pro Met Leu Lys
           35                  40                  45
   Lys Leu Asp Leu Ala Val Ala Asn Ile Thr Lys Glu Glu Asp Leu Asp
       50                  55                  60
   Ala Ile Gly Ser Leu Glu Glu Leu Val Ser Leu Glu Leu Glu Leu Asp
   65                  70                  75                  80
   Asp Thr Ser Ser Gly Ile Glu Arg Ile Val Ser Ser Ser Lys Leu Gln
                   85                  90                  95
   Lys Leu Thr Thr Leu Val Val Lys Val Pro Ser Leu Arg Glu Ile Glu
               100                 105                 110
   Gly Leu Glu Glu Leu Lys Ser Leu Gln Asp Leu Tyr Leu Glu Gly Cys
           115                 120                 125
   Thr Ser Leu Gly Arg Leu Pro Leu Glu Lys Leu Lys Glu Leu Asp Ile
       130                 135                 140
   Gly Gly Cys
   145

## Claims

1. An isolated nucleic acid molecule comprising a sequence of nucleotides corresponding to the nucleotide sequence set forth in SEQ ID NO: 1 or having at least 45% identity thereto and which confers or otherwise facilitates rust resistance in plants, or a sequence of nucleotides complementary thereto.

2. The isolated nucleic acid molecule according to claim 1, comprising a sequence of nucleotides encoding or complementary to a sequence of nucleotides encoding the amino acid sequence set forth in SEQ ID NOS: 2 to 5 or having at least 60% amino acid sequence similarity thereto.

3. The isolated nucleic acid molecule according to claim 1 or claim 2, comprising a sequence of nucleotides encoding or complementary to a sequence encoding a peptide, polypeptide or protein which is capable of interacting with an avirulence gene product of a flax rust.

4. The isolated nucleic acid molecule according to claim 3, wherein the nucleotide sequence corresponds to an allele of the rust resistance gene L or M.

5. The isolated nucleic acid molecule according to claim 4, wherein the rust resistance allele is the L6 allele.

6. The isolated nucleic acid molecule according to claim 4, wherein the rust resistance allele is the L2 allele.

7. The isolated nucleic acid molecule according to claim 4, wherein the rust resistance allele is the L10 allele.

8. An isolated nucleic acid molecule which confers or otherwise facilitates rust resistance in a plant and comprises a nucleotide sequence that is capable of hybridizing under medium stringency conditions to the nucleotide sequence defined in SEQ ID NO: 1 or to the complement of SEQ ID NO: 1.

9. The isolated nucleic acid molecule according to claim 8, which comprises a nucleotide sequence that is capable of hybridizing under high stringency conditions to the nucleotide sequence defined in SEQ ID NO: 1 or to the complement of SEQ ID NO: 1.

10. The isolated nucleic acid molecule according to claim 8, having a nucleotide sequence corresponding to the nucleotide sequence set forth in SEQ ID NO: 1 or having at least 80% identity thereto.

11. The isolated nucleic acid molecule according to any one of claims 1 to 10, wherein said molecule is isolatable from a plant selected from the group consisting of flax, soyabean, sunflower, and any *Linum* species.

12. The isolated nucleic acid molecule according to any one of claims 1 to 11, wherein said molecule is isolatable from a cereal plant.

13. The isolated nucleic acid molecule of claim 12, wherein the cereal plant is wheat or barley or maize, or a wild variety of wheat or barley or maize.

14. A method for identifying a rust resistance-conferring genetic sequence in a plant, said method comprising contacting genomic DNA or cDNA or mRNA from said plant with a hybridizing effective amount of a nucleic acid molecule comprising a genetic sequence conferring or facilitating rust resistance as defined in any one of claims 1 to 13, and then detecting said hybridization.

15. The method according to claim 14, wherein the plant is selected from the group consisting of flax, soyabean, sunflower, and any *Linum* species.

16. The method according to claim 14, wherein the plant is a cereal plant.

17. The method according to claim 16 wherein the cereal plant is wheat or barley or maize, or a wild variety of wheat or barley or maize.

18. The method according to any one of claims 14 to 17, wherein the rust resistance genetic sequence comprises the nucleotide sequence as set forth in SEQ ID NO: 1 or a part thereof encoding rust resistance.

19. The method according to any one of claims 14 to 17, wherein the rust resistance genetic sequence encodes an amino acid sequence substantially as set forth in any one of SEQ ID NOS: 2 to 5 or having at least 60% amino acid sequence similarity thereto.

20. An isolated nucleic acid molecule comprising a sequence of nucleotides which:
(i) confers or otherwise facilitates rust resistance in a plant; and
(ii) is at least 45% identical to the nucleotide sequence of SEQ ID NO: 1 or hybridizes to said sequence under medium stringency conditions; and
(iii) encodes a product having at least one leucine rich region and/or a p-loop comprising the amino acid sequence
wherein X is any amino acid residue, and
wherein the said p-loop is encoded by a sequence at the 5' end of said nucleic acid molecule.

21. The isolated nucleic acid molecule according to claim 20 wherein the product has a leucine rich region which is a leucine rich repeat encoded by the 3' end of the molecule.

22. The isolated nucleic acid molecule according to claim 20 or 21 wherein the product has a leucine rich region which comprises the amino acid sequence: or a sequence having at least 60% similarity thereto.

23. The isolated nucleic acid molecule according to claim 20 wherein the product has a p-loop sequence which comprises the amino acid sequence:

24. The isolated nucleic acid molecule according to claim 23 wherein the p-loop comprises the amino acid sequence GLYGMGGIGKTT.

25. The isolated nucleic acid molecule according to any one of claims 20 to 24 wherein the plant is a cereal plant.

26. The isolated nucleic acid molecule of claim 25 wherein said cereal plant is wheat or barley or maize, or a wild variety of wheat or barley or maize.

27. The isolated nucleic acid molecule according to any one of claims 20 to 24, wherein the plant is a plant selected from the group consisting of flax, soyabean, sunflower, and any *Linum* species.

28. An isolated nucleic acid molecule comprising a modular resistance gene, comprising genetic sequences from at least two nucleic acid molecules encoding disease resistance in plants and wherein at least one of said nucleic acid molecules is the nucleic acid molecule of any one of claims 1 to 13 or claims 20 to 27.

29. The isolated nucleic acid molecule according to claim 28, wherein the modular disease resistance or modular rust resistance gene comprises a nucleotide sequence that encodes a leucine rich region.

30. The isolated nucleic acid molecule according to claim 29 wherein the leucine rich region is encoded by an allele of the rust resistance gene L or M.

31. The isolated nucleic acid molecule of claim 30, wherein the rust resistance allele is selected from the group consisting of L6, L2 and L10.

32. A transgenic plant carrying a non-indigenous genetic sequence comprising the isolated nucleic acid molecule according to any one of claims 1 to 13 or claims 20 to 31.

33. A transgenic plant according to claim 32, wherein said plant is wheat or barley or flax.

34. Use of the isolated nucleic acid molecule according to any one of claims 1 to 13 or claims 20 to 31 to produce a rust-resistant plant, wherein said plant carries said nucleic acid molecule.

35. A method of preparing a transgenic plant having rust resistance comprising introducing into said plant an isolated nucleic acid molecule according to any one of claims 1 to 13 or claims 20 to 31, wherein said plant carries said nucleic acid molecule.

## Patentansprüche

1. Isoliertes Nucleinsäuremolekül, das eine Sequenz von Nucleotiden umfasst, die der in SEQ ID NO: 1 angegebenen Nucleotidsequenz entspricht oder zu mindestens 45 % mit dieser identisch ist, und das Rostbeständigkeit in Pflanzen verleiht oder in anderer Weise ermöglicht, oder eine Sequenz von Nucleotiden, die hierzu komplementär ist.

2. Isoliertes Nucleinsäuremolekül nach Anspruch 1, das eine Sequenz von Nucleotiden mit Codierung für die Aminosäuresequenz, die in SEQ ID NO: 2 bis 5 angegeben ist oder eine zu mindestens 60 % hierzu ähnliche Aminosäuresequenz aufweist, umfasst oder komplementär zu einer derartigen Sequenz von Nucleotiden ist.

3. Isoliertes Nucleinsäuremolekül nach Anspruch 1 oder Anspruch 2, das eine Sequenz von Nucleotiden mit Codierung für ein Peptid, Polypeptid oder Protein, das zur Wechselwirkung mit einem Avirulenzgenprodukt eines Leinrosts fähig ist, umfasst oder komplementär zu einer derartigen Sequenz ist.

4. Isoliertes Nucleinsäuremolekül nach Anspruch 3, wobei die Nucleotidsequenz einem Allel des Rostbeständigkeitsgens L oder M entspricht.

5. Isoliertes Nucleinsäuremolekül nach Anspruch 4, wobei das Rostbeständigkeitsallel das L6-Allel ist.

6. Isoliertes Nucleinsäuremolekül nach Anspruch 4, wobei das Rostbeständigkeitsallel das L2-Allel ist.

7. Isoliertes Nucleinsäuremolekül nach Anspruch 4, wobei das Rostbeständigkeitsallel das L10-Allel ist.

8. Isoliertes Nucleinsäuremolekül, das Rostbeständigkeit in einer Pflanze verleiht oder in anderer Weise ermöglicht und eine Nucleotidsequenz umfasst, die zu einer Hybridisierung unter mittelstringenten Bedingungen mit der in SEQ ID NO: 1 definierten Nucleotidsequenz oder dem Komplement von SEQ ID NO: 1 fähig ist.

9. Isoliertes Nucleinsäuremolekül nach Anspruch 8, das eine Nucleotidsequenz umfasst, die zur Hybridisierung unter hochstringenten Bedingungen mit der in SEQ ID NO: 1 definierten Nucleotidsequenz oder dem Komplement von SEQ ID NO: 1 fähig ist.

10. Isoliertes Nucleinsäuremolekül nach Anspruch 8, das eine Nucleotidsequenz aufweist, die der in SEQ ID NO: 1 angegebenen Nucleotidsequenz entspricht oder zu mindestens 80 % mit dieser identisch ist.

11. Isoliertes Nucleinsäuremolekül nach einem der Ansprüche 1 bis 10, wobei das Molekül aus einer Pflanze, die aus der aus Lein, Sojabohne, Sonnenblume und beliebigen *Linum*-Arten bestehenden Gruppe ausgewählt ist, isolierbar ist.

12. Isoliertes Nucleinsäuremolekül nach einem der Ansprüche 1 bis 11, wobei das Molekül aus einer Getreidepflanze isolierbar ist.

13. Isoliertes Nucleinsäuremolekül nach Anspruch 12, wobei die Getreidepflanze Weizen oder Gerste oder Mais oder eine Wildsorte von Weizen oder Gerste oder Mais ist.

14. Verfahren zur Identifizierung einer Rostbeständigkeit verleihenden Gensequenz in einer Pflanze, wobei das Verfahren das Kontaktieren von genomischer DNA oder cDNA oder mRNA von der Pflanze mit einer zur Hybridisierung wirksamen Menge eines Nucleinsäuremoleküls, das eine wie in einem der Ansprüche 1 bis 13 definierte, Rostbeständigkeit verleihende oder ermöglichende Gensequenz umfasst, und das anschließende Detektieren der Hybridisierung umfasst.

15. Verfahren nach Anspruch 14, wobei die Pflanze aus der aus Lein, Sojabohne, Sonnenblume und beliebigen *Linum*-Arten bestehenden Gruppe ausgewählt ist.

16. Verfahren nach Anspruch 14, wobei die Pflanze eine Getreidepflanze ist.

17. Verfahren nach Anspruch 16, wobei die Getreidepflanze Weizen oder Gerste oder Mais oder eine Wildsorte von Weizen oder Gerste oder Mais ist.

18. Verfahren nach einem der Ansprüche 14 bis 17, wobei die Rostbeständigkeitsgensequenz die in SEQ ID NO: 1 angegebene Nucleotidsequenz oder einen Teil derselben mit Codierung für Rostbeständigkeit umfasst.

19. Verfahren nach einem der Ansprüche 14 bis 17, wobei die Rostbeständigkeitsgensequenz eine Aminosäuresequenz codiert, die im wesentlichen einer der Sequenzen SEQ ID NO: 2 bis 5 angegebenen entspricht oder eine zu mindestens 60 % hierzu ähnliche Aminosäuresequenz aufweist.

20. Isoliertes Nucleinsäuremolekül, das eine Sequenz von Nucleotiden umfasst, die:
(i) Rostbeständigkeit in einer Pflanze verleiht oder in anderer Weise ermöglicht; und
(ii) zu mindestens 45 % identisch mit der Nucleotidsequenz von SEQ ID NO: 1 ist oder mit der Sequenz unter mittelstringenten Bedingungen hybridisiert; und
(iii) ein Produkt codiert, das mindestens eine leucinreiche Region und/oder eine p-Schleife, die die Aminosäuresequenz
worin X ein beliebiger Aminosäurerest ist, und wobei die p-Schleife durch eine Sequenz am 5'-Ende des Nucleinsäuremoleküls codiert wird, umfasst, aufweist.

21. Isoliertes Nucleinsäuremolekül nach Anspruch 20, wobei das Produkt eine leucinreiche Region aufweist, die ein durch das 3'-Ende des Moleküls codiertes leucinreiches Repeat ist.

22. Isoliertes Nucleinsäuremolekül nach Anspruch 20 oder 21, wobei das Produkt eine leucinreiche Region aufweist, die die Aminosäuresequenz: oder eine Sequenz, die zu mindestens 60 % zu dieser ähnlich ist, umfasst.

23. Isoliertes Nucleinsäuremolekül nach Anspruch 20, wobei das Produkt eine p-Schleife-Sequenz aufweist, die die Aminosäuresequenz umfasst.

24. Isoliertes Nucleinsäuremolekül nach Anspruch 23, wobei die p-Schleife die Aminosäuresequenz GLYGMGGIGKTT umfasst.

25. Isoliertes Nucleinsäuremolekül nach einem der Ansprüche 20 bis 24, wobei die Pflanze eine Getreidepflanze ist.

26. Isoliertes Nucleinsäuremolekül nach Anspruch 25, wobei die Getreidepflanze Weizen oder Gerste oder Mais oder eine Wildsorte von Weizen oder Gerste oder Mais ist.

27. Isoliertes Nucleinsäuremolekül nach einem der Ansprüche 20 bis 24, wobei die Pflanze eine aus der aus Lein, Sojabohne, Sonnenblume und beliebigen *Linum*-Arten bestehenden Gruppe ausgewählte Pflanze ist.

28. Isoliertes Nucleinsäuremolekül, das ein modulares Resistenzgen, das Gensequenzen von mindestens zwei Nucleinsäuremolekülen mit Codierung für Krankheitsresistenz in Pflanzen umfasst und wobei mindestens eines der Nucleinsäuremoleküle das Nucleinsäuremolekül nach einem der Ansprüche 1 bis 13 oder der Ansprüche 20 bis 27 ist, umfasst.

29. Isoliertes Nucleinsäuremolekül nach Anspruch 28, wobei das modulare Krankheitsresistenz- oder modulare Rostbeständigkeitsgen eine Nucleotidsequenz, die eine leucinreiche Region codiert, umfasst.

30. Isoliertes Nucleinsäuremolekül nach Anspruch 29, wobei die leucinreiche Region durch ein Allel des Rostbeständigkeitsgens L oder M codiert wird.

31. Isoliertes Nucleinsäuremolekül nach Anspruch 30, wobei das Rostbeständigkeitsallel aus der aus L6, L2 und L10 bestehenden Gruppe ausgewählt ist.

32. Transgene Pflanze, die eine Gensequenz, die keine eigene ist, die das isolierte Nucleinsäuremolekül nach einem der Ansprüche 1 bis 13 oder der Ansprüche 20 bis 31 umfasst, trägt.

33. Transgene Pflanze nach Anspruch 32, wobei die Pflanze Weizen oder Gerste oder Lein ist.

34. Verwendung des isolierten Nucleinsäuremoleküls nach einem der Ansprüche 1 bis 13 oder der Ansprüche 20 bis 31 zur Herstellung einer rostbeständigen Pflanze, wobei die Pflanze das Nucleinsäuremolekül trägt.

35. Verfahren zur Herstellung einer transgenen Pflanze mit Rostbeständigkeit, das das Einführen eines isolierten Nucleinsäuremoleküls nach einem der Ansprüche 1 bis 13 oder der Ansprüche 20 bis 31 in die Pflanze umfasst, wobei die Pflanze das Nucleinsäuremolekül trägt.

## Revendications

1. Molécule d'acide nucléique isolée comprenant une séquence de nucléotides correspondant à la séquence nucléotidique présenté dans SEQ ID NO: 1 ou ayant une identité d'au moins 45 % avec elle, et qui confère aux plantes, ou leur facilite d'une autre manière, une résistance à la rouille, ou une séquence de nucléotides complémentaire de celle-ci.

2. Molécule d'acide nucléique isolée selon la revendication 1, comprenant une séquence de nucléotides codant, ou complémentaire d'une séquence nucléotidique codant la séquence d'acides aminés présentée dans SEQ ID NOS: 2 à 5 ou ayant une similitude de séquence d'acides aminés d'au moins 60 % avec elle.

3. Molécule d'acide nucléique isolée selon la revendication 1 ou la revendication 2, comprenant une séquence de nucléotides codant, ou complémentaire d'une séquence nucléotidique codant, un peptide, un polypeptide ou une protéine qui est capable d'interagir avec un produit de gène d'avirulence d'une rouille du lin.

4. Molécule d'acide nucléique isolée selon la revendication 3, dans laquelle la séquence nucléotidique correspond à un allèle du gène de résistance à la rouille L ou M.

5. Molécule d'acide nucléique isolée selon la revendication 4, dans laquelle l'allèle de résistance à la rouille est l'allèle L6.

6. Molécule d'acide nucléique isolée selon la revendication 4, dans laquelle l'allèle de résistance à la rouille est l'allèle L2.

7. Molécule d'acide nucléique isolée selon la revendication 4, dans laquelle l'allèle de résistance à la rouille est l'allèle L10.

8. Molécule d'acide nucléique isolée qui confère à une plante, ou lui facilite d'une autre manière, une résistance à la rouille, et comprend une séquence nucléotidique qui est capable de s'hybrider dans des conditions de stringence moyenne à la séquence nucléotidique définie dans SEQ ID NO: 1 ou au complément de SEQ ID NO: 1.

9. Molécule d'acide nucléique isolée selon la revendication 8, qui comprend une séquence nucléotidique qui est capable de s'hybrider dans des conditions de stringence élevée à la séquence nucléotidique définie dans SEQ ID NO: 1 ou au complément de SEQ ID NO: 1.

10. Molécule d'acide nucléique isolée selon la revendication 8, ayant une séquence nucléotidique correspondant à la séquence nucléotidique présenté dans SEQ ID NO: 1 ou ayant une identité d'au moins 80 % avec elle.

11. Molécule d'acide nucléique isolée selon l'une quelconque des revendications 1 à 10, ladite molécule pouvant être isolée à partir d'une plante choisie dans le groupe constitué du lin, du soja, du tournesol et de toutes les espèces de *Linum.*

12. Molécule d'acide nucléique isolée selon l'une quelconque des revendications 1 à 11, ladite molécule pouvant être isolée à partir d'une plante céréalière.

13. Molécule d'acide nucléique isolée selon la revendication 12, laquelle plante céréalière étant le blé, l'orge ou le maïs, ou une variété sauvage de blé, d'orge ou de maïs.

14. Procédé d'identification d'une séquence génétique conférant une résistance à la rouille dans une plante, ledit procédé comprenant le fait de mettre en contact un ADN génomique ou un ADNc ou un ARNm provenant de ladite plante avec une quantité efficace pour l'hybridation d'une molécule d'acide nucléique comprenant une séquence génétique conférant ou facilitant la résistance à la rouille telle que définie dans l'une quelconque des revendications 1 à 13, et de détecter ensuite ladite hybridation.

15. Procédé selon la revendication 14, dans lequel la plante est choisie dans le groupe constitué du lin, du soja, du tournesol et de toutes les espèces de *Linum.*

16. Procédé selon la revendication 14, dans lequel la plante est une plante céréalière.

17. Procédé selon la revendication 16, dans lequel la plante céréalière est le blé, l'orge ou le maïs, ou une variété sauvage de blé, d'orge ou de maïs.

18. Procédé selon l'une quelconque des revendications 14 à 17, dans lequel la séquence génétique de résistance à la rouille comprend la séquence nucléotidique présentée dans SEQ ID NO: 1 ou l'une de ses parties codant une résistance à la rouille.

19. Procédé selon l'une quelconque des revendications 14 à 17, dans lequel la séquence génétique de résistance à la rouille code une séquence d'acides aminés sensiblement telle que présentée dans l'une quelconque des SEQ ID NOS: 2 à 5 ou ayant une similitude de séquence d'au moins 60 % avec elles.

20. Molécule d'acide nucléique isolée comprenant une séquence de nucléotides qui :
(i) confère à une plante, ou lui facilite d'une autre manière, une résistance à la rouille ; et
(ii) est identique à au moins 45 % à la séquence de SEQ ID NO: 1 ou s'hybride à ladite séquence dans des conditions de stringence moyenne ; et
(iii) code un produit ayant au moins une région riche en leucine et/ou une boucle p comprenant la séquence d'acides aminés
dans laquelle X est un résidu d'acide aminé quelconque, et dans laquelle ladite boucle p est codée par une séquence à l'extrémité 5' de ladite molécule d'acide nucléide.

21. Molécule d'acide nucléique isolée selon la revendication 20, dans laquelle le produit a une région riche en leucine qui est une répétition riche en leucine codée par l'extrémité 3' de la molécule.

22. Molécule d'acide nucléique isolée selon la revendication 20 ou 21, dans laquelle le produit a une région riche en leucine qui comprend la séquence d'acides aminés : ou une séquence ayant une similitude d'au moins 80 % avec elle.

23. Molécule d'acide nucléique isolée selon la revendication 20, dans laquelle le produit a une séquence de boucle p qui comprend la séquence d'acides aminés :

24. Molécule d'acide nucléique isolée selon la revendication 23, dans laquelle la boucle p comprend la séquence d'acides aminés GLYGMGGIGKTT

25. Molécule d'acide nucléique isolée selon l'une quelconque des revendications 20 à 24, laquelle plante étant une plante céréalière.

26. Molécule d'acide nucléique isolée selon la revendication 25, ladite plante céréalière étant le blé, l'orge ou le maïs, ou une variété sauvage de blé, d'orge ou de maïs.

27. Molécule d'acide nucléique isolée selon l'une quelconque des revendications 20 à 24, laquelle plante étant une plante choisie dans le groupe constitué du lin, du soja, du tournesol et de toutes les espèces de *Linum.*

28. Molécule d'acide nucléique isolée comprenant un gène de résistance modulaire, comprenant des séquences génétiques issues d'au moins deux molécules d'acide nucléique codant une résistance à une maladie dans des plantes et dans laquelle au moins une desdites molécules d'acide nucléique est la molécule d'acide nucléique de l'une quelconque des revendications 1 à 13 ou des revendications 20 à 27.

29. Molécule d'acide nucléique isolée selon la revendication 28, dans laquelle le gène de résistance à une maladie modulaire ou de résistance à la rouille modulaire comprend une séquence nucléotidique qui code une région riche en leucine.

30. Molécule d'acide nucléique isolée selon la revendication 29, dans laquelle la région riche en leucine est codée par un allèle du gène de résistance à la rouille L ou M.

31. Molécule d'acide nucléique isolée de la revendication 30, dans laquelle l'allèle de résistance à la rouille est choisi dans le groupe consistant en L6, L2 et L10.

32. Plante transgénique portant une séquence génétique non indigène comprenant la molécule d'acide nucléique isolée selon l'une quelconque des revendications 1 à 13 ou des revendications 20 à 31.

33. Plante transgénique selon la revendication 32, ladite plante étant le blé ou l'orge ou le lin.

34. Utilisation de la molécule d'acide nucléique isolée selon l'une quelconque des revendications 1 à 13 ou des revendications 20 ou 31 pour produire une plante résistante à la rouille, dans laquelle ladite plante porte ladite séquence d'acide nucléique.'

35. Procédé de préparation d'une plante transgénique ayant une résistance à la rouille comprenant le fait d'introduire dans ladite plante d'une molécule d'acide nucléique isolée selon l'une quelconque des revendications 1 à 13 ou des revendications 20 à 31, dans lequel ladite plante porte ladite molécule d'acide nucléique.
